(19) **Europäisches Patentamt / European Patent Office / Office européen des brevets**

(11) **EP 4 534 137 A1**

(12) **EUROPEAN PATENT APPLICATION**
published in accordance with Art. 153(4) EPC

(43) Date of publication:
09.04.2025 Bulletin 2025/15

(21) Application number: 23811171.0

(22) Date of filing: 26.05.2023

(51) International Patent Classification (IPC):
*A61N 1/362* (2006.01)

(52) Cooperative Patent Classification (CPC):
A61B 5/352; A61N 1/362; A61N 1/365

(86) International application number:
PCT/CN2023/096504

(87) International publication number:
WO 2023/227101 (30.11.2023 Gazette 2023/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.05.2022 CN 202210593496

(71) Applicant: United Innomed (Shanghai) Limited
Shanghai 201315 (CN)

(72) Inventor: WANG, Li
Shanghai 201315 (CN)

(74) Representative: Banse & Steglich
Patentanwälte PartmbB
Patentanwaltskanzlei
Herzog-Heinrich-Straße 23
80336 München (DE)

(54) **IMPLANTABLE HEART FAILURE TREATMENT DEVICE**

(57) Provided is an implantable heart failure treatment device, including a housing; an anchoring member, connected to the housing and fixing the housing to a heart; a pair of stimulation electrodes coupled to the housing, configured to be in contact with a predetermined stimulation position of the heart, and configured to apply an electrical stimulation pulse for enhancing cardiac contractility to the predetermined stimulation position; a pulse generation module accommodated within the housing, electrically coupled to the stimulation electrodes, and configured to generate the electrical stimulation pulse; and a control module accommodated within the housing, electrically coupled to the stimulation electrodes and the pulse generation module, and configured to: receive a far-field sense signal indicating a global excitation event of the heart of a patient and a local sense signal indicating a local excitation event of a local position of the heart of the patient; and determine, at least based on the far-field sense signal and the local sense signal, whether the local excitation event of the local position of the heart of the patient corresponds to a specific global excitation event, and transmit the electrical stimulation pulse to the stimulation electrodes in an absolute refractory period of the local excitation event.

FIG. 1

EP 4 534 137 A1

## Description

FIELD OF THE INVENTION

The present application relates to the field of heart failure treatment, and in particular, to an implantable heart failure treatment device and a method for treating a heart failure based on the device.

BACKGROUND

Cardiac contractility modulation (CCM) is a unique and innovative therapy for treating chronic heart failure patients. It enhances cardiomyocyte contractility by sending an electrical stimulation to the myocardium, thereby achieving treatment goals. Unlike traditional pacemakers or cardiac resynchronization therapy devices, a CCM device sends an electrical stimulation in an absolute refractory period, which will not affect the heart rate or an action potential distribution. The existing CCM device on the market typically includes a main unit device implanted in a part outside the heart and two bipolar leads extending into the inter-ventricular septum of the ventricles and configured to sense electrical activities of the local myocardium and a time sequence of the electrical activities, thereby determining a deliverable time period for sending an electrical pulse stimulation to the myocardium, and applying the electrical pulse stimulation to local cardiac muscle cells in the deliverable time period to enhance the cardiomyocyte contractility.

However, the two bipolar leads of the existing CCM device implanted in the body not only affect normal blood flows in blood vessels and of the right heart and closure of the tricuspid valve, but may also fall off, and further cause infections (including capsules, electrode wires, and the like), thus resulting in additional safety risks. Meanwhile, the existing CCM device determines deliverable time of the electrical pulse stimulation based on an electrical signal of a local myocardial position, which may lead to a misjudgment on the deliverable time. For example, if the CCM device confirms that the electrical signal of the local myocardial position corresponds to an R wave of a cardiac cycle, but in fact the signal corresponds to a T wave or another event (e.g. an interference signal), the electrical pulse stimulation applied by the CCM device will act on the myocardium in the T wave phase. This may significantly increase the risk of inducing malignant ventricular arrhythmia including ventricular tachycardia (VT) or ventricular fibrillation (VF).

In addition, under the same energy, a longer distance between stimulation electrodes or a larger effective area of each stimulation electrode has a higher level or a larger range of possible impact on the far-field myocardium. If a local pulse stimulation occurs at a part where cardiac muscle cells are depolarized relatively later, time of applying the electrical pulse stimulation may fall within an exaltation phase of an action potential of cardiac muscle cells at a myocardial position which are depolarized earlier, which may lead to further depolarization of this part of myocardium. To avoid the aforementioned problems, the stimulation electrodes of the existing CCM device need to be placed in the inter-ventricular septum, making it unsuitable for other myocardial positions that possibly require enhancement of the contractility. Moreover, to avoid the above-mentioned risks, a control strategy for the existing CCM device is not applying the electrical pulse stimulation when a non-atrial-induced (ventricular ectopic excitation) myocardial potential changes. However, premature ventricular contractions have a high incidence rate among heart failure patients. Some heart failure patients rely on ventricular pacing for long time, especially for patients who take beta blockers for long time, patients with single-chamber implantable cardioverter-defibrillators (ICDs), and patients in cardiac resynchronization therapy (CRT). Therefore, the therapeutic effect of the existing CCM device on these patients will be greatly reduced.

In summary, it is necessary to provide a new heart failure treatment device and method to address at least one of the issues in the existing technology.

SUMMARY

One objective of the present application is to provide an implantable heart failure treatment device and a method for treating a heart failure based on the device.

In a first aspect of the present application, an implantable heart failure treatment device is provided. The device includes: a housing; an anchoring member connected to the housing and configured to fix the housing to a heart of a patient; a pair of stimulation electrodes coupled to the housing, configured to be in contact with a predetermined stimulation position of the heart of the patient, and configured to sense a local sense signal generated by discharging of cardiac muscle cells at the predetermined stimulation position and apply an electrical stimulation pulse for enhancing cardiomyocyte contractility of the heart of the patient to the predetermined stimulation position; a pulse generation module accommodated within the housing, electrically coupled to the pair of stimulation electrodes, and configured to generate the electrical stimulation pulse; and a control module, accommodated within the housing, electrically coupled to the pair of stimulation electrodes and the pulse generation module, and configured to: receive a far-field sense signal indicating a global excitation event of the heart of the patient; determine, at least based on the far-field sense signal and the local sense signal, whether a local excitation event of the cardiac muscle cells at the predetermined stimulation position corresponds to a specific global excitation event; and transmit, when it is determined that the local excitation event corresponds to the specific global excitation event, the electrical stimulation pulse to the pair of stimulation electrodes during an absolute refractory period of the local excitation event.

In a second aspect of the present application, an implantable heart failure treatment device is further provided. The device includes: a housing; an anchoring member connected to the housing and configured to fix the housing to a heart of a patient; a pair of stimulation electrodes coupled to the housing, configured to be in contact with a predetermined stimulation position of the heart of the patient, and configured to apply, to the predetermined stimulation position an electrical stimulation pulse for enhancing cardiomyocyte contractility of the heart of the patient and an electrical pacing pulse for adjusting a heart rate of the heart of the patient; a pulse generation module, accommodated within the housing, electrically coupled to the pair of stimulation electrodes, and configured to generate the electrical stimulation pulse and the electrical pacing pulse; and a control module accommodated within the housing, electrically coupled to the pair of stimulation electrodes and the pulse generation module, and configured to receive a far-field sense signal indicating a global excitation event of the heart of the patient, determine, at least based on the far-field sense signal and the applied electrical pacing pulse, whether a local excitation event of cardiac muscle cells at the predetermined stimulation position corresponds to a specific global excitation event, and transmit, when it is determined that the local excitation event corresponds to the specific global excitation event, the electrical stimulation pulse to the pair of stimulation electrodes during an absolute refractory period of the local excitation event.

In a third aspect of the present application, an implantable heart failure treatment device is further provided. The device includes: a housing; an anchoring member connected to the housing and configured to fix the housing to a heart of a patient; a pair of stimulation electrodes coupled to the housing, configured to be in contact with a predetermined stimulation position of the heart of the patient, and configured to apply an electrical stimulation pulse for enhancing cardiomyocyte contractility of the heart of the patient to the predetermined stimulation position; a pulse generation module, accommodated within the housing, electrically coupled to the pair of stimulation electrodes, and configured to generate the electrical stimulation pulse; and a control module, accommodated within the housing, electrically coupled to the pair of stimulation electrodes and the pulse generation module, and configured to: receive a far-field sense signal indicating a global excitation event of the heart of the patient; determine, at least based on the far-field sense signal and local sense signal, a pulse delivery time and a pulse deliverable time window; and transmit, when the pulse delivery time falls into the pulse deliverable time window, the electrical stimulation pulse to the pair of stimulation electrodes.

In a fourth aspect of the present application, an implantable heart failure treatment device is further provided. The device includes: a housing; an anchoring member connected to the housing and configured to fix the housing to a heart of a patient; a pair of stimulation electrodes coupled to the housing, configured to be in contact with a predetermined stimulation position of the heart of the patient, and configured to apply an electrical stimulation pulse for enhancing cardiomyocyte contractility of the heart of the patient to the predetermined stimulation position; a pulse generation module accommodated within the housing, electrically coupled to the pair of stimulation electrodes, and configured to generate the electrical stimulation pulse; and a control module accommodated within the housing, electrically coupled to the pair of stimulation electrodes and the pulse generation module, and configured to: receive a far-field sense signal indicating a global excitation event of the heart of the patient and a local sense signal indicating a local excitation event of a local position of the heart of the patient; determine, at least based on the far-field sense signal and the local sense signal, whether the local excitation event of the local position of the heart of the patient corresponds to a specific global excitation event; and transmit, when it is determined that the local excitation event corresponds to the specific global excitation event, the electrical stimulation pulse to the pair of stimulation electrodes during an absolute refractory period of the local excitation event.

In a fifth aspect of the present application, a method for treating a heart failure based on an implantable device is further provided. The implantable device includes a housing; the housing is fixed to the heart of a patient through an anchoring member on the housing; and the implantable device further includes a pair of stimulation electrodes configured to be in contact with a predetermined stimulation position of a heart of the patient. The method includes: sensing, by the pair of stimulation electrodes, a local sense signal generated by discharging of cardiac muscle cells at the predetermined stimulation position; receiving a far-field sense signal indicating a global excitation event of the heart of the patient; determining, at least based on the far-field sense signal and the local sense signal, whether a local excitation event of the cardiac muscle cells at the predetermined stimulation position corresponds to a specific global excitation event; and when it is determined that the local excitation event corresponds to the specific global excitation event, applying the electrical stimulation pulse to the predetermined stimulation position by using the pair of stimulation electrodes during an absolute refractory period of the local excitation event.

In a sixth aspect of the present application, a method for treating a heart failure based on an implantable device is further provided. The implantable device includes a housing; the housing is fixed to the heart of a patient through an anchoring member on the housing; and the implantable device further includes a pair of stimulation electrodes configured to be in contact with a predetermined stimulation position of a heart of the patient. The method includes: applying, by the pair of stimulation electrodes, an electrical pacing pulse for adjusting a heart rate of the heart of the patient to the predetermined stimulation position of the heart of the patient; determining capture of the heart of the patient; after the capture has been determined, determining, based on an obtained far-field sense signal indicating a global excitation event of the heart of the patient and the electrical pacing pulse, whether a local excitation event of cardiac muscle cells at the predetermined stimulation position corresponds to a specific global excitation event; and when it is determined that the local excitation event corresponds to the specific global excitation event, applying the electrical stimulation pulse to the predetermined stimulation position by using the pair of stimulation electrodes during an absolute refractory period of the local excitation event.

In a seventh aspect, the present disclosure further provides a computer-readable storage medium having a computer program stored thereon. The computer program, when executed by a processor, implements steps of the method in any one of the fifth aspect and the sixth aspect of the present application.

The above is an overview of the present application, which may be simplified, summarized, or omitted in detail. Those skilled in the art should recognize that this section is only illustrative and not intended to limit the scope of the present application in any way. This overview section is neither intended to identify the key or essential features of the claimed subject matter, nor is intended to serve as an auxiliary means for determining the scope of the claimed subject matter.

BRIEF DESCRIPTION OF DRAWINGS

The above and other features of the content of the present application will be understood more fully and clearly through the following specification and attached claims and in conjunction with the accompanying drawings. It can be understood that these accompanying drawings only depict several embodiments of the present application and should not be considered as limiting the scope of the content of the present application. By using the accompanying drawings, the content of the present application will be explained more clearly and in detail.

FIG. 1 is a schematic diagram of an implantable heart failure treatment device fixed within a ventricle according to an embodiment of the present application;
FIG. 2A to FIG. 2C respectively show schematic diagrams of implantable heart failure treatment devices according to three different embodiments of the present application;
FIG. 3A to FIG. 3C respectively show schematic diagrams of implantable heart failure treatment devices according to three different embodiments of the present application;
FIG. 4A to FIG. 4C respectively show schematic diagrams of implantable heart failure treatment devices according to three different embodiments of the present application;
FIG. 5A to FIG. 5B respectively show schematic diagrams of implantable heart failure treatment devices according to two different embodiments of the present application;
FIG. 6 is a flowchart of a control method for an implantable heart failure treatment device according to an embodiment of the present application;
FIG. 7 is a working flowchart of an implantable device in a pacemaker mode according to an embodiment of the present application;
FIG. 8 is a schematic diagram of modules of a control system for an implantable heart failure treatment device according to an embodiment of the present application;
FIG. 9 is a flowchart of a pulse stimulation control method according to Embodiment 1 of the present invention;
FIG. 10 is a first flowchart of a pulse stimulation control method according to Embodiment 2 of the present invention;
FIG. 11 is a second flowchart of a pulse stimulation control method according to Embodiment 2 of the present invention;
FIG. 12 is a third flowchart of a pulse stimulation control method according to Embodiment 2 of the present invention;
FIG. 13 is a fourth flowchart of a pulse stimulation control method according to Embodiment 2 of the present invention;
FIG. 14 is a fifth flowchart of a pulse stimulation control method according to Embodiment 2 of the present invention;
FIG. 15 is a schematic electrocardiogram corresponding to R wave sensing with a single electrode lead according to Embodiment 2 of the present invention;
FIG. 16 is a schematic electrocardiogram corresponding to R wave sensing with a plurality of electrode leads according to Embodiment 2 of the present invention;
FIG. 17 is a schematic diagram of modules of a pulse stimulation control system according to Embodiment 3 of the present invention;
FIG. 18 is a schematic diagram of modules of a pulse stimulation control system according to Embodiment 4 of the present invention; and
FIG. 19 is a schematic structural diagram of an electronic device for implementing a pulse stimulation control method according to Embodiment 5 of the present invention.

DETAILED DESCRIPTION

The following detailed description has been referred to in the accompanying drawings that form a portion of the description. In the accompanying drawings, similar symbols usually represent similar constitutes, unless otherwise specified in the context. The illustrative implementations described in the detailed description, accompanying drawings, and claims are not intended to be limitative. Without departing from the spirit or scope of the subject matter of the present application, other implementations may be employed and other changes may be made. It can be understood that various configurations, substitutions, combinations, and designs can be made to the various aspects of the content of the present application generally described in the present application and illustrated in the accompanying drawings, and all these clearly constitute a portion of the content of the present application.

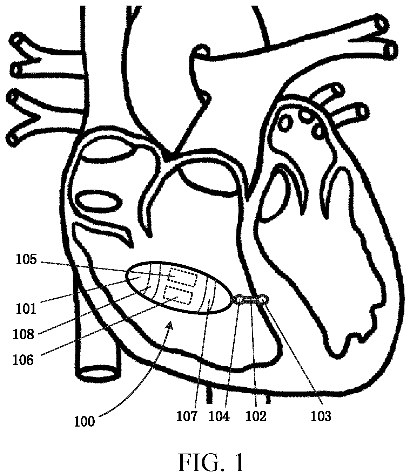

FIG. 1 is a schematic diagram of an implantable heart failure treatment device 100 fixed within a ventricle according to an embodiment of the present application. The implantable heart failure device 100 can be implanted into the body in various suitable ways, such as transapical or transvascular implantation.

As shown in FIG. 1, the implantable heart failure treatment device 100 includes a housing 101 and an anchoring member 102 connected to the housing 101. The anchoring member 102 is configured to fix the housing 100 at a specific position in the heart of a patient. Although not specifically shown in the figures, the anchoring member 102 may include various suitable mechanical components such as a pin portion, a nail portion, a screw portion, a hook portion, a thread portion, a spiral portion, sharp teeth, a clamping portion, and/or other similar structures, to fix the anchoring member to heart tissues. In some embodiments, one or more anchoring members 102 may have hook-shaped portions that can pierce through the heart tissues and at least partially remain in the heart tissues. In other embodiments, one or more anchoring members 102 may have twist drill structures similar to a wine opener, so that the anchoring member(s) 102 can drill into the heart tissues and achieve fixation. In still some other embodiments, one or more anchoring members 102 may have screw portions, thereby achieving close engagement with the heart tissues through screw rotation. A person skilled in the art can understand that the structures of the anchoring members 102 mentioned above are only exemplary and not restrictive.

Although a single anchoring member 102 is shown in FIG. 1, the implantable heart failure treatment device 100 may have any suitable number of anchoring members 102 to fix the housing 101 to the heart tissue. For example, device 100 may include one, two, three, four, five, six, seven, eight, or more anchoring members 102. Meanwhile, the implantable heart failure treatment device 100 may alternatively adopt a combination of various anchoring members mentioned above to achieve a more stable fixing effect.

Still referring to FIG. 1, the implantable heart failure treatment device 100 further includes a pair of stimulation electrodes coupled to the housing 100 and composed of electrodes 103 and 104. The electrode 103 or 104 may include one or more biocompatible conductive materials, such as various metals or alloys known to be safely implanted in a human body. The pair of stimulation electrodes is exposed to a surrounding tissue and/or blood that the stimulation electrodes are in contact with, so that the stimulation electrodes can send an electrical signal to or receive an electrical signal from the surrounding tissue and/or blood. The electrical signal can be transmitted through the heart tissues and/or blood. In some embodiments, the pair of stimulation electrodes 103 and 104 is configured to sense a local sense (LS) signal generated by discharging of cardiac muscle cells at a predetermined stimulation position, and apply an electrical stimulation pulse for enhancing cardiomyocyte contractility of the heart of the patient to the predetermined stimulation position.

Since the anchoring member 102 shown in FIG. 1 is implanted at an inter-ventricular septum position, the pair of stimulation electrodes 103 and 104 can apply the electrical stimulation to the inter-ventricular septum position nearby. However, depending on the position of the anchoring member 102, the predetermined stimulation position where the pair of stimulation electrodes 103 and 104 is located can be any other heart position where it is desired to apply the electrical stimulation pulse for enhancing the cardiomyocyte contractility. For example, the predetermined stimulation position can be a position on the endocardium inside the cardiac chamber of the patient or on the epicardium of the heart of the patient. In some embodiments, the predetermined stimulation position corresponds to a portion of a ventricular tissue. In some other embodiments, the predetermined stimulation position may alternatively be a portion of an atrial tissue. In some embodiments, the housing 101 of the device 100 is configured to be suitable for being placed in a vein on an outer side of the heart of the patient (e.g. with a capsule-like structure), and its predetermined stimulation position is an epicardial tissue near the vein. In this case, the anchoring member 102 is constructed as a suitable structure. For example, the anchoring member 102 may be a stent, a balloon, a supporting arm, or a similar expandable structure, or the anchoring member 102 may be an anchor or a claw that extends out of the housing 101 and is in contact with the vascular wall of the vein. In still some embodiments, the anchoring member 102 may be composed of a portion of the housing 101. A diameter of a cross section of the anchoring member 102 is set to be connected to a relatively narrow segment of the vascular wall of the vein, thereby fixing the housing 101 in the vein.

As shown in FIG. 1, the implantable heart failure treatment device 100 further includes a pulse generation module 105 and a control module 106 which are arranged within the housing 101 and are electrically coupled to the pair of stimulation electrodes 103 and 104. Although the positions of the electrodes 103 and 104 as shown in the figure are both located on the anchoring member 102, they may be any structures that can be electrically coupled to the pulse generation module 105 and the control module 106 inside the housing 101 and are exposed to tissues and/or blood outside the housing 101. For example, at least one of the pair of electrodes 103 and 104 is not arranged on the anchoring member 102. In some embodiments, the electrode 103 and/or 104 may be formed on an outer surface of the housing or arranged on another member extending out of the housing. When the anchoring member 102 is fixed to the heart or a vascular tissue, the electrode 103 and/or 104 remain in contact with the heart or the vascular tissue. For example, the anchoring member 102 may have the hook-like structure as previously described, and the electrode 103 and/or 104 are located on a protrusion extending out of the housing and adjacent to the anchoring member 102. When the hook-shaped portion of the anchoring member 102 pierces through the heart tissue and is at least partially accommodated within the heart tissue, the protrusion where the electrode 103 and/or 104 is located is in contact with the heart tissue. In some other embodiments, the electrode 103 and/or 104 may be arranged on the anchoring member 102 or formed by the entirety or a portion of the anchoring member 102, and be inserted into the heart or vascular tissue along with the anchoring member 102. For example, the anchoring member 102 may have the foregoing twist drill structure similar to a wine opener. A portion or the entirety of the twist drill constitutes the electrode 103 and/or the 104, and the electrode 103 and/or 104 are at least partially drilled into the heart tissue.

In some embodiments, the pair of stimulation electrodes 103 and 104 can alternatively use any electrode configuration of a traditional leadless pacemaker for applying a pacing pulse. In some embodiments, the electrode 103 and/or 104 may be defined by a portion of the housing 101. For example, a surface of the housing 101 includes an insulating material, and a portion not covered by the insulating material constitutes the electrode 103 and/or 104. The electrode 103 and/or 104 may be formed by a region of any shape, having an area smaller than a surface area of the housing, such as a circular region, a rectangular region, an annular region, a semi-annular region, a fan-shaped region, and an arch region. In some embodiments, the electrode 103 and/or 104 is configured to be partially located in the housing 101 and partially exposed out of a surface of the housing 101. In still some embodiments, the electrode 103 and/or 104 is arranged on another member extending out of the housing 101 and is spaced apart from the housing 101 and/or the anchoring member 102. It should be emphasized that the above description is not intended to limit the specific position or construction of the electrode 103 and/or 104. The electrode 103 and/or 104 can use any combination of the above electrode positions and structural form features, or any feasible position arrangement or structural form that can achieve its function.

As shown in FIG. 1, the electrode 103 in the pair of stimulation electrodes is configured to be in contact with or electrically connected to the predetermined stimulation position (the inter-ventricular septum position in the figure) of the cardiac muscle tissue. The electrode 103 and/or 104 itself may have various available sizes and/or shapes. In some embodiments, a surface area of the electrode 103 for being in contact with the predetermined stimulation position is 1 to 10 mm$^2$, preferably 1.5, 2, 3, 4, 5 mm$^2$. In the pair of stimulation electrodes, the electrode 103 and the electrode 104 are separated from each other. In some embodiments, a distance between the two electrodes is 2 to 20 mm, preferably 5 to 10 mm.

Continuing with reference to FIG. 1, in addition to sensing a local electrical signal generated by the discharging of the cardiac muscle cells at the predetermined stimulation position, the implantable heart failure treatment device 100 may further include a far-field excitation sense module, which is configured to sense a global excitation event of the heart of the patient, such as a P wave which represents time and a potential change of depolarization of an atrial global muscle group, a QRS wave complex which represents time and a potential change of depolarization of a ventricular global muscle group, a T wave which represents time and a potential change of repolarization of the ventricular global muscle group. After obtaining a Far-field Sense (also called Global Sense, GS for short) signal associated with the global excitation event, the far-field excitation sense module provides the corresponding far-field sense signal to a control module 106 of the implantable heart failure treatment device 100, such that the control module 106 can further determine specific delivery time of a stimulation pulse based on the far-field sense signal and the local sense signal.

As shown in FIG. 1, the far-field excitation sense module includes a pair of sensing electrodes composed of sensing electrodes 107 and 108. The pair of sensing electrodes is coupled to the housing 101 and is configured to sense a far-field electrical signal involved in the above global excitation event. It should be noted that although the electrodes 107 and 108 shown in FIG. 1 are arranged on an outer surface of the housing 101, they can have any structure that is electrically coupled to the control module 106 and exposed to tissues and/or blood outside the housing 101. In other words, the sensing electrodes 107 and 108 may use any combination of electrode materials and structural form features used in the stimulation electrodes 103 and 104 as described above, or any feasible position arrangement or structural form that can achieve their functions. In some embodiments, a surface area of the sensing electrode 107 and/or 108 is larger than that of the stimulation electrode 103 and/or 104. In some embodiments, the electrode 107 and/or 108 is defined by a portion of the housing 101, and a surface area of the electrode(s) occupies a quarter to one-third of the overall surface area of the housing. In some embodiments, the pair of stimulation electrodes and the pair of sensing electrodes can share one electrode. For example, the sense electrode 107 and the stimulation electrode 104 can be combined into one electrode. A detailed arrangement of the sensing electrodes and the stimulation electrodes will be elaborated as the following.

Although not shown in the figure, in some embodiments, the far-field excitation sense module may alternatively be arranged outside the housing 101 and is communicably connected to the control module 106. For example, the far-field excitation sense module can be configured in another implantable device independent of the implantable heart failure treatment device 100 and be communicably connected to the implantable heart failure treatment device 100 in a wireless or wired manner. The far-field excitation sense module arranged in the another implantable device can use the form of the pair of sensing electrodes 107 and 108 as described above to obtain a far-field sense signal and send the far-field sense signal or a processing result of the far-field sense signal to the control module 106 of the implantable heart failure treatment device 100. The another implantable device may be any available implantable device with a sensing and/or processing function for the far-field sense signal.

In some embodiments, two or more implantable heart failure treatment devices may jointly form a heart failure treatment system, and at least one of the implantable heart failure treatment devices does not have a far-field excitation sense module, but obtains a far-field sense signal from the far-field excitation sense module of another implantable heart failure treatment device in a wired or wireless manner. In some embodiments, the aforementioned heart failure treatment system includes the implantable heart failure treatment device 100 disposed in the right ventricle as shown in FIG. 1, and another implantable heart failure treatment device disposed within the coronary vein of the heart and communicably connected to the device 100. Compared with the device 100, the implantable heart failure treatment device in the coronary vein may not have the pair of sensing electrodes 107 and 108. Furthermore, the control module of the implantable heart failure treatment device within the coronary vein is configured to sense a local sense signal generated by discharging of cardiac muscle cells at a predetermined stimulation position through the stimulation electrodes, and send the local sense signal and/or a processing result of the local sense signal to the device 100. Subsequently, the device 100 determines, based on the above local sense signal and the far-field sense signal obtained by the device 100, specific time of applying an electrical stimulation pulse, and sends, to the device within the coronary vein, an instruction of applying an electrical stimulation pulse. After receiving the instruction, the device applies an electrical stimulation to the predetermined stimulation position. In some other embodiments, the device within the coronary vein may be configured to only obtain the far-field sense signal indicating the global excitation event of the heart of the patient from the device 100, and determine, based on the far-field sense signal and the local sense signal, specific time of applying an electrical stimulation pulse, and apply an electrical stimulation pulse. A detailed method for determining, based on the far-field sense signal and the local sense signal, the time of applying an electrical stimulation pulse will be elaborated as the following.

In some other embodiments, the far-field excitation sense module may include a pair of sensing electrode patches configured to be in contact with the body surface of the patient to sense a body surface electrocardiogram signal of the patient as a far-field sense signal, and transmit, in a wired or wireless manner, the signal to the control module 106 of the implantable heart failure treatment device 100 fixed to the heart. Since the far-field sense signal reflects a far-field characteristic of the activity of the heart of the patient, sharing the far-field sense signal can effectively reduce the manufacturing cost of the device. Of course, in some embodiments, the far-field sense signal indicating the global excitation event of the heart of the patient that received by the control module 106 of the implantable heart failure treatment device 100 may alternatively be from any other device communicably connected to the control module 106 that has a function of sensing and/or processing the far-field sense signal. These devices can be any other type of implantable device placed inside the body of the patient, or any type of device placed on the body surface or outside the body.

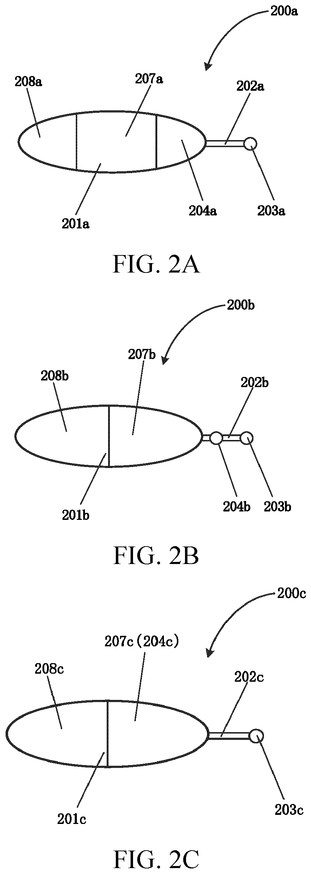

In the housing 101, the pulse generation module 105 is electrically coupled to the pair of stimulation electrodes 103 and 104 and is configured to generate an electrical stimulation pulse. The control module 106 is electrically coupled to the pair of stimulation electrodes 103 and 104 and the pulse generation module 105, and is configured to receive the local sense signal generated by the discharging of the cardiac muscle cells at the predetermined stimulation position which is obtained through the pair of stimulation electrodes 103 and 104, and the far-field sense signal indicating the global excitation event of the heart of the patient. Meanwhile, the control module 106 further determines, at least based on the far-field sense signal and the local sense signal, whether the local excitation event of the cardiac muscle cells at the predetermined stimulation position corresponds to a specific global excitation event, for example, determines whether an electrical signal comprehensively generated by action potentials of cardiac muscle cell groups at and near the predetermined stimulation position corresponds to a specific far-field electrocardiogram change. When it is determined that the local excitation event corresponds to the specific global excitation event, during an absolute refractory period of the local excitation event, the control module 106 controls the pulse generation module 105 to transmit the electrical stimulation pulse to the pair of stimulation electrodes 103 and 104, thereby enhancing the cardiomyocyte contractility of the cardiac muscle cells during the local excitation event, to treat a heart failure. A detailed method of how the control module 106 determines, based on the above signals, an instruction for transmitting the electrical stimulation pulse will be elaborated as the following. FIGS. 2A to 2C respectively show schematic diagrams of implantable heart failure treatment devices 200a, 200b, and 200c according to three different embodiments of the present application, schematically showing alternative arrangements of a pair of sensing electrodes and a pair of stimulation electrodes relative to a housing.

As shown in FIG. 2A, an electrode 203a of a pair of stimulation electrodes in the device 200a is located at one end of an anchoring member 202a away from a housing 201a, and the other electrode 204a is arranged at one end of the housing 201a close to the anchoring member 202a. The pair of sensing electrodes 207a and 208a of the device 200a are arranged on the housing 201a in sequence in a direction away from the electrode 204a. The arrangement of a pair of sensing electrodes and a pair of stimulation electrodes of the device 200b shown in FIG. 2B is generally the same as the embodiment shown in FIG. 2A, except that a stimulation electrode 204b is arranged on an anchoring member 202b, instead of a housing 201b. In addition, sensing electrodes 207a and 207b of the device 200b are arranged at two ends of the housing 201b. The only difference between the implantable heart failure treatment device 200c shown in FIG. 2C and the implantable heart failure treatment device 200b shown in FIG. 2B is that the stimulation electrode 204b arranged on the anchoring member 202b is canceled, but an electrode 207c at one end of a housing 201c close to an anchoring member 202c is used as a common reference potential electrode for the pair of sensing electrodes and the pair of stimulation electrodes. In other words, in the device 200c, the pair of sensing electrodes is composed of electrodes 207c and 208c, and the pair of stimulation electrodes is composed of electrodes 203c and 207c.

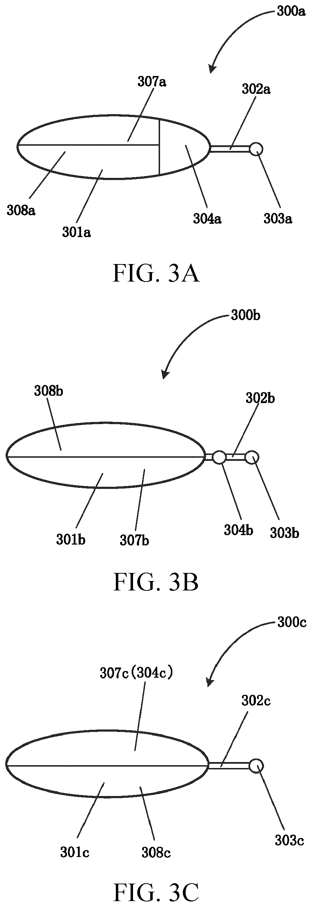

FIG. 3A to FIG. 3C respectively show schematic diagrams of implantable heart failure treatment devices 300a, 300b, and 300c according to different embodiments of the present application, schematically showing position arrangements of a pair of sensing electrodes and a pair of stimulation electrodes relative to a housing. The structure of the implantable heart failure treatment device 300a shown in FIG. 3A is generally the same as that of the implantable heart failure treatment device 200a shown in FIG. 2A, except for the arrangement direction of the electrodes of the pair of sensing electrodes. If the arrangement direction of the sensing electrodes 207a and 208a shown in FIG. 2A is defined as a long axis direction of the housing, a pair of sensing electrodes 307a and 308a shown in FIG. 3A is arranged in a direction generally perpendicular to the long axis direction. Similarly, the structure of the implantable heart failure treatment device 300b shown in FIG. 3B is generally the same as that of the implantable heart failure treatment device 200b shown in FIG. 2B, except that a pair of sensing electrodes 307b and 308b is arranged along a housing 301b in a direction generally perpendicular to the long axis direction. The structure of the implantable heart failure treatment device 300c shown in FIG. 3C is generally the same as that of the implantable heart failure treatment device 200c shown in FIG. 2C, except that a pair of sensing electrodes 307c and 308c is arranged along a housing 301c in a direction generally perpendicular to the long axis direction.

In some embodiments, a distance between the sensing electrodes of the pair of sensing electrodes is set in a relatively distant manner. For example, the sensing electrodes are arranged at two ends of a long axis of the housing, on two surfaces of a short axis which are far from each other, or on two surfaces of the two ends of the long axis of the housing, which can better acquire electrical activities of the entire heart, especially the electrical activities of the entire ventricular muscle. Specifically, in some embodiments, if one electrode of the pair of stimulation electrodes is arranged on the housing, and the pair of far-field sensing electrodes does not share this stimulation electrode, a region covered by the pair of stimulation electrodes and a region covered by the pair of sensing electrodes do not overlap. Assuming that the pair of sensing electrodes includes electrodes A and B, and the pair of stimulation electrodes includes electrodes C and D, D being an electrode in contact with the myocardium, an arrangement order of the electrodes is A, B, C, and D. In some instances, an insulator may be provided between electrodes B and C to reserve a distance between the two electrodes, thereby minimizing the impact of a pulse stimulation delivered by the stimulation electrodes on the electrical activities sensed by the sensing electrodes. A person skilled in the art can understand that regardless of the setting, any two of the electrodes A, B, C, and D need to be insulated from each other.

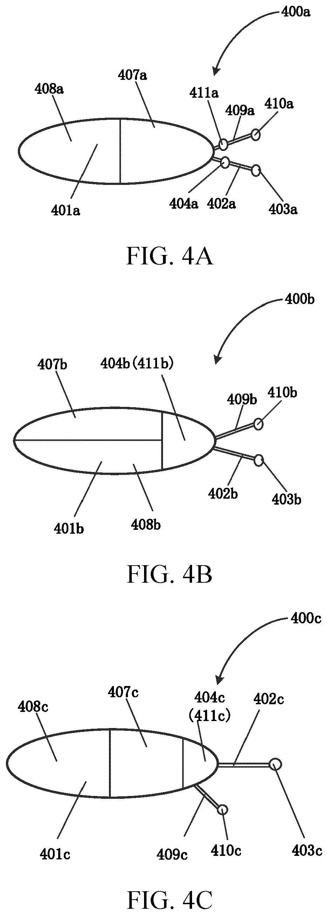

FIGS. 4A to 4C respectively show schematic diagrams of implantable heart failure treatment devices 400a, 400b, and 400c according to different embodiments of the present application. These implantable heart failure treatment devices all include a plurality of pairs of stimulation electrodes. The structure of the implantable heart failure treatment device 400a shown in FIG. 4A is generally the same as that of the implantable heart failure treatment device 200b shown in FIG. 2B, except that the device 400a further includes another anchoring member 409a. Another pair of stimulation electrodes 410a and 411a is arranged on the anchoring member 409a. The two pairs of stimulation electrodes are configured to be respectively in contact with different predetermined stimulation positions on the endocardium or the epicardium. These pairs of stimulation electrodes can sense a local sense (LS) signal generated by action potentials of cardiac muscle cells at the corresponding predetermined stimulation positions, and can further apply electrical stimulation pulses for enhancing cardiomyocyte contractility of the heart of the patient to the corresponding predetermined stimulation positions.

In some embodiments, assuming that an electrode 403a of a pair of stimulation electrodes 403a and 404a is in contact with a position A within the ventricular lumen, and an electrode 410a of the pair of stimulation electrodes 410a and 411a is in contact with a position B within the ventricular lumen that is different from the position A. The two electrodes respectively sense a local sense signal generated by membrane potential changes of cardiac muscle cells at the position A and the position B. When the local sense signals at the position A and the position B satisfy a preset condition, an electrical stimulation pulse is applied to the position A and the position B through the pair of stimulation electrodes to enhance the cardiomyocyte contractility. For example, the control module of the device 400a can determine, based on the intensities and/or a chronological order of the local sense signals at the position A and the position B, whether electrical activities of the cardiac muscle cells at the position A and/or the position B correspond to a depolarization process of the ventricle; and applies, when a determination result is "yes", an electrical stimulation pulse to cardiac muscle tissues at the position A and/or the position B.

The implantable heart failure treatment device 400b in the embodiment shown in FIG. 4B is generally the same as the implantable heart failure treatment device 300a in FIG. 3A, except that the device 400b further includes another anchoring member 409b, and another stimulation electrode 410b arranged on the anchoring member 409b. An electrode 404b composed of a portion of a housing 401b forms two pairs of stimulation electrodes with electrodes 403b and 410b, respectively. The structure of the device 400c shown in FIG. 4C is generally the same as that of the device 200a shown in FIG. 2A, except that the device 400c further includes another anchoring member 409c, and an electrode 404c composed of a portion of a housing 401c forms two pair of stimulation electrodes with electrodes 403c and 410c, respectively. Similar to the description of the device 400a in FIG. 4A, the devices 400b and 400c can be configured to sense and process local sense signals at different positions of the heart and deliver electrical stimulation pulses separately through the two pairs of stimulation electrodes, which will not be elaborated here.

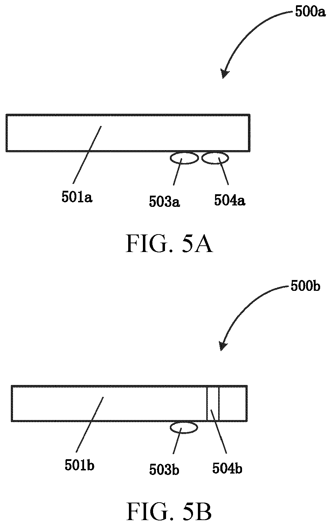

FIG. 5A to FIG. 5B respectively show schematic diagrams of implantable heart failure treatment devices 500a and 500b according to different embodiments of the present application, schematically showing the position arrangements of a pair of stimulation electrodes relative to a housing. As shown in FIG. 5A, the device 500 includes a housing 501a and a pair of stimulation electrodes composed of electrodes 503a and 504a attached to the housing 501a. As mentioned earlier, the device 500 is designed to be placed within a vein on an outer side of the heart of a patient, and a predetermined stimulation position of the device is an epicardial cardiac muscle tissue near the vein. The pair of stimulation electrodes is configured to sense a local sense (LS) signal generated by discharging of cardiac muscle cells at a specific position on the epicardium, and can further apply an electrical stimulation pulse for enhancing cardiomyocyte contractility of the heart of the patient to the corresponding position. As shown in the figure, the housing 501a and the pair of stimulation electrodes attached to a surface of the housing are constructed to be able to be connected to the vascular wall of the vein on the outer side of the heart, to fix the device 500a in the vein, and at least one electrode of the pair of stimulation electrodes is configured to abut against one side of the vascular wall close to the epicardium.

In addition, in some embodiments, the device 500a may further include a stent, a balloon, a supporting arm, or a similar expandable structure, or an anchoring member including an anchor or claw structure, so as to be connected to the vascular wall of the vein to fix the device 500. In some other embodiments, a size of at least a portion of the housing 501a is configured to be fixed in a relatively narrow section of a vein on the outer side of the heart, thereby fixing the housing 501a within the vein. The implantable heart failure treatment device 500b shown in FIG. 5B is generally the same as the device 500a in FIG. 5A, except that the structure of a single electrode 504b in a pair of stimulation electrodes has been replaced with a portion of the housing 501a, e.g. an annular region, which is not covered by an insulating material, on the housing 501a. In some embodiments, a single electrode 504b may be a local region in the annular region facing the myocardium, e.g. a semi-ring facing the myocardium, or an arc smaller or larger than a semi-ring. As mentioned above, two or more implantable heart failure treatment devices disclosed in the present application may jointly form a heart failure treatment system, and at least one of the implantable heart failure treatment devices may not have far-field excitation sensing electrodes and a far-field excitation sense module, but obtains a far-field sense signal and/or a signal processing result from the far-field excitation sense module of another implantable heart failure treatment device in a wired or wireless manner. The implantable heart failure treatment devices 500a and 500b shown in FIG. 5A or 5B may not have a far-field excitation sense module to minimize its volume to facilitate its placement within the vein on the outer side of the heart. However, the devices can receive a far-field sense signal indicating global excitation events of the heart of the patient from other extracorporeal devices or other implanted devices with far-field excitation sense modules; and determine by the control modules of the devices, based on a local sense signal sensed by the pair of stimulation electrodes of the devices, whether local excitation events of cardiac muscle cells at corresponding predetermined stimulation positions correspond to specific global excitation events. In this way, when the local excitation events satisfy a predetermined condition, the implantable heart failure treatment device can instruct the pair of stimulation electrodes to apply the electrical stimulation pulses to the predetermined stimulation positions. In some embodiments, the implantable heart failure treatment devices 500a and 500b may be configured to not perform the aforementioned determining step based on the far-field sense signal and the local sense signal, and communicate with other implanted devices or extracorporeal devices in the system, to send the local sense signals or sense results obtained by the pair of stimulation electrodes to other implanted or extracorporeal devices in the system. In this way, other implanted or extracorporeal devices can make a judgment based on the far-field sense signal and the local sense signal; and send, to the implantable heart failure treatment device 500a or 500b at appropriate time, an instruction for delivering an electrical stimulation pulse. Later, the device 500a or 500b controls, based on the received instruction, the pair of stimulation electrodes to apply the electrical stimulation pulse to the predetermined stimulation position (epicardium) that is in contact with or corresponds to the pair of stimulation electrodes, so as to enhance the cardiomyocyte contractility.

It should be emphasized that the embodiments shown in FIGS. 2A to 5B are intended to illustrate the possible position arrangements of the pair of stimulation electrodes and/or the pair of sensing electrodes. The materials, structural forms, and the like of the pair of stimulation electrodes and/or pair of sensing electrodes in the figures can use any technical features and combinations thereof described for the electrodes 103, 104, 107, and 108 described in the embodiment shown in FIG. 1, or use any other feasible position arrangement or structural form that can achieve the functions. This will not be elaborated here. In addition, for the sake of simplifying the view, some views do not specifically depict insulating materials or structures between the electrodes, but insulating structures or materials are obviously arranged between the electrodes. For example, for a plurality of electrodes arranged on the housing, the electrodes can be isolated through insulating material coatings or insulating material warppings. For example, the housing of the device can be entirely made of an insulating material, and two or more electrodes can extend out of the housing and be connected to a circuit module inside the housing. A person skilled in the art can clearly understand that the insulation between these electrodes can be implemented in any feasible way, which will not be elaborated here.

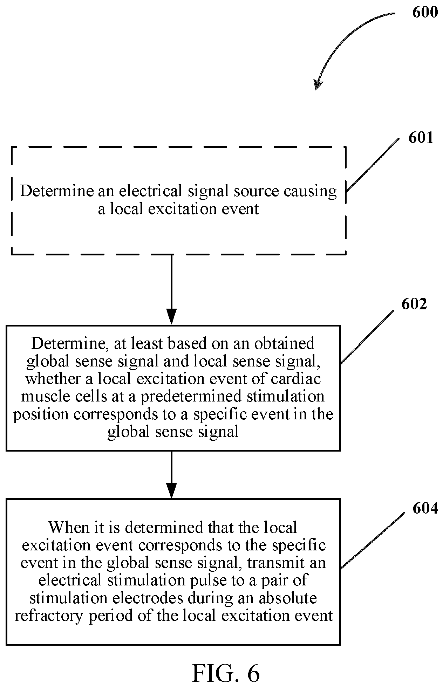

FIG. 6 shows a flowchart of a control method 600 for an implantable device according to an embodiment of the present application. Steps of the method shown in the figure will be exemplarily illustrated below in conjunction with the device 100 shown in FIG. 1.

In step 602, the control module 106 determines, at least based on an obtained far-field sense signal and a local sense signal, whether a local excitation event of cardiac muscle cells at a predetermined stimulation position corresponds to a specific global excitation event. In some embodiments, step 602 may include: obtaining a specific event time window corresponding to the specific global excitation event; and if time of sensing the local excitation event falls into the specific event time window, it is determined that the local excitation event corresponds to the specific global excitation event.

The specific event time window is a time period determined based on the far-field sense signal (also referred to as GS signal herein) and/or the local sense signal (also referred to as LS signal herein) and associated with a specific event of a cardiac cycle. For example, the time period associated with the specific event (e.g. an R wave or a QRS wave complex) of the cardiac cycle can be determined based on far-field electrocardiogram signals sensed by the sensing electrodes of one or more implanted devices or surface electrocardiograms sensed by one or more extracorporeal devices. In some embodiments, in a case that the implantable heart failure treatment device includes a plurality of pairs of stimulation electrodes, the specific event time window can be determined based on a plurality of LS signals provided by the pairs of stimulation electrodes at different predetermined stimulation positions.

Specifically, a start point of the specific event time window can be determined by the GS signal and/or LS signal mentioned above. In some embodiments, the start point of the specific event time window may be determined based on a time point at which the specific event is sensed in the far-field sense signal (hereinafter referred to as the "GS sensing time") and/or a time point at which the local sense signal generated by the discharging of the cardiac muscle cells at the predetermined stimulation position is sensed (hereinafter referred to as the "LS sensing time").

In the embodiment of the heart failure treatment device implanted in the right ventricle as shown in FIG. 1, the start point of the specific event time window (GVT-s for short in the following formulas) can be set in the following way:

When the LS sensing time is later than the GS sensing time, namely, when a difference between the LS sensing time and the GS sensing time (GLSD for short in the following formulas) is greater than 0, the GVT-s is set to a time point from a preset time length (A in the following formulas) before the GS sensing time (GS for short in the following formulas), a specific formula of which is as follows:

when GLSD>0, GVT-s=GS-A.

On the contrary, when the LS sensing time is earlier than or equal to the GS sensing time, the GVT-s can be set to a time point from the preset time length before the LS sensing time (LS for short in the following formulas), a specific formula of which is as follows:

when $H \leq GLSD \leq 0$, GVT-s=LS-A. This formula can a be transformed to GVT-s=GS+GLSD-A, where H<0, preferably -120 ms $\leq$ H $\leq$ -20 ms. During actual sensing of an electrocardiogram signal, a delay in capturing the electrocardiogram signal may cause the GS sensing time to be later than a real time node, or for other reasons, cause the LS sensing time to be slightly earlier than the GS sensing time. Since LS is used as start time, this situation can be better addressed, and the correspondence between a local excitation event and a global excitation event can be more accurately determined.

The above settings can be implemented in real time in each cardiac cycle.

It should be further noted that by monitoring the LS signal and the GS signal within a presetting period of parameters, the implantable heart failure treatment device may determine a reference time difference B between the LS sensing time and the GS sensing time in a case that the LS sensing time is later than the GS sensing time, and determine a reference time difference C between the LS sensing time and the GS sensing time in a case that the LS sensing time is earlier than or equal to the GS sensing time. In a subsequent operation process, the GVT-s can be set based on the above reference time difference and the LS sensing time or the GS sensing time, a specific formula of which is as follows:

when GLSD>0, GVT-s=LS-B-A;
when $H \leq GLSD \leq 0$, GVT-s=GS-C-A; H<0, preferably, $-120 \text{ ms} \leq H \leq -20 \text{ ms}$.

It should be noted that the above specific calculation methods are only illustrative examples, and the start point of the specific event time window may alternatively be determined using other methods or based on other parameters. In the above embodiments, the preset time lengths A, B, and C can all be adjustable preset values, where A can be selected from 0 to 40 ms, preferably 20 ms. In some embodiments, the preset time lengths B and C may use the same preset value.

A length of the specific event time window can be a preset time length which is usually related to a specific type of a specific event. An R wave being a specific event is taken as an example. For an R wave caused by a self-generated impulse at the atrial position (e.g. generated by the atrionector), the length of the specific event time window (or referred to as an R wave time window) can be set to 30 to 130 ms, preferably 90 ms. For an R wave caused by self-generated activities at the ventricular position (e.g. premature ventricular contraction) or an external electrical stimulation to the ventricular tissues, the length of the R wave time window can be set to 30 to 250 ms, preferably 150 ms.

In some other examples, the length related to a ventricular self-generated electrical activity and a ventricular electrical activity caused by pacemaking can be set separately by a doctor according to an actual situation. The time length can be set differently for different premature ventricular contractions by a doctor according to specific situations.

Still referring to FIG. 6, in step 604, the control module 106 transmits an electrical stimulation pulse to the pair of stimulation electrodes during an absolute refractory period of the local excitation event when it is determined that the local excitation event corresponds to the specific global excitation event.

In some embodiments, step 604 specifically includes: determining a pulse delivery time by the control module 106 based on the local sense signal and/or the far-field sense signal; determining, based on the local sense signal and/or the far-field sense signal, a pulse deliverable time window suitable for transmitting an electrical stimulation pulse; and when the pulse delivery time falls into the pulse deliverable time window, transmitting the electrical stimulation pulse to the pair of stimulation electrodes 103 and 104, so as to enhance the cardiomyocyte contractility.

In the present application, the pulse delivery time (CSDT for short herein) means a time point at which the pair of stimulation electrodes delivers the electrical stimulation pulse, and the time point is determined based on the obtained GS signal and/or LS signal. The pulse delivery time can be determined in different ways. The setting of the CSDT ensures that the delivery time of pulse stimulation is within the absolute refractory period of the local excitation event.

In some embodiments, the pulse delivery time can be determined based on the LS sensing time. For example, the pulse delivery time can be time from the preset time length after the LS sensing time, a specific formula of which is as follows:

$$CSDT=LS+D.$$

In some other embodiments, the pulse delivery time is determined based on the combination of the GS sensing time and the GLSD. For example, the pulse delivery time can be a sum of the GS sensing time, the GLSD, and the preset time length, a specific formula of which is as follows:

$$CSDT=GS+GLSD+D.$$

As mentioned earlier, by sensing the LS signal and the GS signal within a presetting period, a reference time difference E between the LS sensing time and the GS sensing time can be determined in a case that the LS sensing time is later than the GS sensing time; and a reference time difference F between the LS sensing time and the GS sensing time can be determined in a case that the LS sensing time is earlier than or equal to the GS sensing time. In a subsequent operation process, the CSDT can be determined based on the above reference time difference and the LS sensing time or the GS sensing time, a specific formula of which is as follows:

when GLSD>0, CSDT=GS+E+D;
when $H \leq GLSD \leq 0$, GSDT=GS-F+D, where H<0, preferably $-120 \leq H \leq -20$. During actual sensing of an electrocardiogram signal, a delay in capturing the electrocardiogram signal may cause the GS sensing time to be later than a real time point, or for other reasons, cause the LS sensing time to be slightly earlier than the GS sensing time. Since LS is used as start time, this situation can be better addressed, and the correspondence between a local excitation event and a global excitation event can be more accurately determined.

E and F above can be determined in real time in each cardiac cycle, or can be determined as preset values during a setting period.

It should be noted that the above specific calculation methods are only illustrative examples, and the pulse delivery time may alternatively be determined using other methods or based on other parameters. In the above embodiments, the preset time length D can be selected from 10 to 80 ms, preferably 40 ms.

The pulse deliverable time window is a time period suitable for delivering an electrical stimulation pulse, during which the transmission of the electrical stimulation pulse through the stimulation electrodes to the predetermined stimulation position will not or will barely cause further depolarization events of cardiac muscle tissues at the predetermined stimulation position and other cardiac muscle tissues that can be affected by the electrical stimulation pulse. Since the pulse deliverable time window is set as a determining condition, the implantable heart failure treatment device can enable the electrical stimulation pulse to be delivered at an appropriate time point within the absolute refractory period of the myocardium at the stimulation position and the myocardium that can be affected by the stimulation pulse, in order to enhance the cardiac contractility and avoid the risk of inducing malignant ventricular arrhythmias such as ventricular tachycardia (VT) or ventricular fibrillation (VF).

In some embodiments, the start point of the pulse deliverable time window can be determined in the same way as the start point of the specific event time window as described above, and will not be elaborated here. In some embodiments, the start points of the specific event time window and the pulse deliverable time window may use any other feasible methods. For example, in the case that the implantable heart failure treatment device includes a plurality of pairs of stimulation electrodes, for example, devices are implanted within both the right ventricle and the left ventricle, the specific event time window and the pulse deliverable time window can be determined based on a GS signal and an LS signal of a pair of stimulation electrodes that senses a local excitation event earliest in a cardiac cycle. Certainly, the pulse deliverable time window can be determined based on an LS signal, a GS signal, and various factors mentioned above.

Like the setting of the length of the specific event time window, the length of the pulse deliverable time window can be a preset time length which is usually related to a specific type of a specific event. An R wave being a specific event is taken as an example. For an R wave caused by a self-generated impulse at the atrial position (e.g. generated by the atrionector), the length of the pulse deliverable time window (or referred to as an R wave pulse deliverable time window) can be set to 30 to 250 ms, preferably 130 ms. For an R wave caused by self-generated activities at the ventricular position or an external electrical stimulation to the ventricular tissues, the length of the pulse deliverable time window can be set to 30 to 250 ms, preferably 200 ms.

In some other examples, the length of the pulse deliverable time window of a self-generated electrical activity at the ventricular position and a ventricular electrical activity caused by ventricular pacemaking can be set separately by a doctor according to an actual situation. Different time lengths of premature ventricular contractions can be set by a doctor according to specific situations.

It should be noted that the start time points corresponding to the pulse deliverable time window and the specific event time window (also referred to as "sensing time window" in some descriptions herein) can be the same or different. In other words, the pulse deliverable time window and the specific event time window can be independently set without any correlation or dependency between each other. Specifically, the pulse deliverable time window and specific event time window can be determined based on a far-field electrocardiogram signal, and can be adaptively adjusted according to factors such as a specific situation of a sensed specific event (e.g. R wave) and a specific position of the myocardium, so as to cover or meet the needs of the majority of pulse stimulation scenarios. The specific determination methods for the pulse deliverable time window and the specific event time window will be described in detail in conjunction with the accompanying drawings and embodiments as the following. Optionally, the method 600 may further include step 601 before step 602. In step 601, an electrical signal source causing a local excitation event is determined. The electrical signal source may include a self-generated impulse from the atrial position, a self-generated ventricular activity from the ventricular position, or a ventricular electrical activity caused by an electrical stimulation from an exterior of the heart. In some embodiments, a specific type of the electrical signal source can be determined through a far-field sense signal. Later, in conjunction with the electrical signal source determined in step 601, the specific event time window, the pulse deliverable time window, the pulse delivery time, and other parameters can be adjusted correspondingly in step 602 and step 604. The specific adjustment methods can be found in the descriptions in step 602 and step 604. In the case where the electrical signal source is the electrical stimulation (e.g. pacing pulse) from the exterior of the heart, the specific adjustment methods can be found in the description of a control method for the implantable heart failure treatment device with a pacing pulse function as the following.

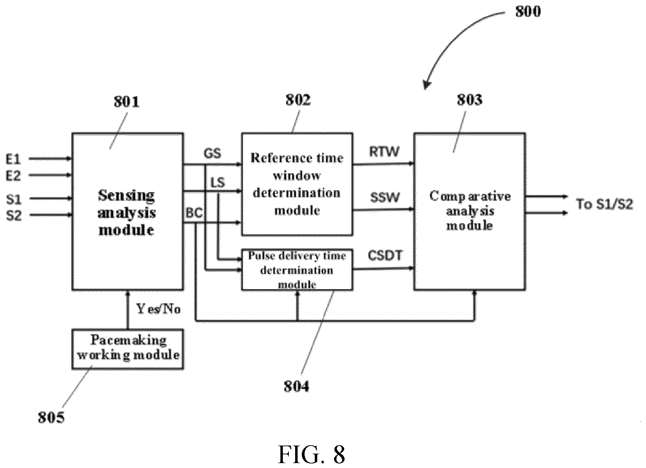

FIG. 8 is a schematic diagram of modules of a control system for an implantable heart failure treatment device 800 according to an embodiment of the present application. As shown in FIG. 8, the device 800 includes a sensing analysis module 801 configured to sense and receive a local sense signal (LS in FIG. 8) and a far-field sense signal (GS in FIG. 8) which are obtained by a pair of stimulation electrodes (S1 and S2 in FIG. 8) and a pair of sensing electrodes (E1 and E2 in FIG. 8) of the device 800, and transmit the signals to a reference time window determination module 802 and a pulse delivery time determination module 804. In some embodiments, the far-field sense signal (GS) includes a far-field electrocardiogram signal of the heart of a patient sensed by the pair of sensing electrodes. The reference time window determination module 802 determines a specific event (e.g. R wave) time window (the R wave time window is represented by RTW in the figure) corresponding to a specific event in the far-field electrocardiogram signal, and/or a pulse deliverable time window (SSW) according to the obtained GS signal and/or LS signal.

Still referring to FIG. 8, the sensing analysis module 801 of the device 800 can be further configured to obtain a signal that represents an electrical activity source causing a local excitation event (BC in FIG. 8), and transmit the signal to the reference time window determination module 802 and the pulse delivery time determination module 804. The reference time window determination module 802 is configured to determine, based on a received BC signal, LS signal, and GS signal, whether a local excitation event of cardiac muscle cells at a predetermined stimulation position corresponds to a specific global excitation event. An R wave being a specific event is taken as an example. In a case that the BC signal indicates, for example, the electrical activity source is an electrical activity spontaneously transmitted from the atrial position, a ventricular self-generated electrical activity from the ventricular position, or a ventricular electrical activity caused by an external electrical stimulation, the reference time window determination module 802 correspondingly sets the specific event time window RTW and the pulse deliverable time window SSW, and sends the RTW and the SSW to a comparative analysis module 803. Similarly, in a case that the BC signal indicates that the electrical signal source is a self-generated impulse from the atrial position, a self-generated impulse from the ventricular position, or an electrical stimulation from an exterior of the heart, the pulse delivery time determination module 804 correspondingly determines pulse discharge time CSDT. Based on the above information, the comparative analysis module 803 determines whether to apply an electrical stimulation pulse to a predetermined stimulation position of the heart of the patient through the pair of stimulation electrodes. In some embodiments, the comparative analysis module 803 first determines whether the time of sensing the local excitation event in the LS signal falls into the RTW. If the time of sensing the local excitation event in the LS signal falls into the RTW, it is determined that the local electrical excitation event at the predetermined stimulation position corresponds to an R wave event caused thereby, and the electrical stimulation pulse can be transmitted to the pair of stimulation electrodes S1 and S2 during an absolute refractory period of the local electrical excitation event. Furthermore, when the comparative analysis module 803 determines that the time of sensing the local excitation event in the LS signal falls into the RTW, the comparative analysis module 803 then determines whether the pulse delivery time CSDT falls into the pulse deliverable time window SSW, and only transmits the electrical stimulation pulse to the pair of stimulation electrodes S1 and S2 if the CSDT falls into the SSW.

As shown in FIG. 8, the device 800 may further include a pacemaking working module 805 which is configured to provide a signal of whether to deliver a pacing pulse to the sensing analysis module 801. After obtaining a signal indicating that the pacing pulse has been delivered, the sensing analysis module 801 determines whether heart capture has been achieved. If the heart capture has been achieved, and the type of the BC signal is pacemaking (e.g. ventricular pacemaking), the reference time window determination module 802 correspondingly sets the specific event time window RTW and the pulse deliverable time window SSW. Meanwhile, the pulse delivery time determination module 804 correspondingly determines the pulse delivery time CSDT. These signals are then sent to the comparative analysis module 803. The comparative analysis module 803 first determines, based on the obtained information, whether applying time of an electrical pacing pulse falls into the RTW. If the applying time of the electrical pacing pulse falls into the RTW, it is determined that the local electrical excitation event at the predetermined stimulation position corresponds to a ventricular muscle electrical activity caused thereby, and a subsequent contraction event. In addition, the electrical stimulation pulse can be transmitted to the pair of stimulation electrodes S1 and S2 during an absolute refractory period of the local electrical excitation event. Furthermore, after determining that the applying time of the pacing pulse falls into the RTW, the comparative analysis module 803 can then determine whether the pulse delivery time CSDT falls into the pulse deliverable time window SSW, and only transmits the electrical stimulation pulse to the pair of stimulation electrodes if the CSDT falls into the SSW. Certainly, in a case that the achievement of the heart capture is determined after the delivery of the pacing pulse, the correspondence between the delivery of the pacing pulse and a specific global excitation event (e.g. R wave) reflected by the far-field sense signal can usually be directly determined. Therefore, in some embodiments, in a case of delivery of the pacing pulse, it is also possible to directly determine whether the local excitation event at the predetermined stimulation position as described above corresponds to the specific global excitation event according to whether the capture has occurred.

It should be noted that in the embodiment of FIG. 8 above, division of the modules is based on functions or execution steps, rather than physical division of the specific modules. In fact, one or more modules can be merged into an entire module, or each module may be further divided. In addition, the steps and functions performed by each module are not necessarily in the order shown in FIG. 8. For example, in some embodiments, the reference time window determination module 802 can first determine whether the occurrence time of the local excitation event sensed in the LS signal falls into the RTW, and then determine, only if the occurrence time falls into the RTW, whether the pulse delivery time CSDT is within the pulse deliverable time window SSW, so as to determine whether to deliver a stimulation pulse. The various modules in the device shown in FIG. 8 can be implemented by hardware, software or firmware, or a combination of hardware and software/firmware. In addition, one or more of the GS and LS signals in the device 800 shown in FIG. 8 may come from other implanted devices or extracorporeal devices, rather than just from the pair of sensing electrodes E1 and E2 and the pair of stimulation electrodes S1 and S2.

In the embodiments described above, the local sense signal generated by the discharging of the cardiac muscle cells at the predetermined stimulation position is used as the local sense signal indicating the local excitation event at the local position of the heart of the patient, and whether the local excitation event of the cardiac muscle cells at the predetermined stimulation position of the heart of the patient corresponds to the specific global excitation event is further determined based on the far-field sense signal indicating the global excitation event of the heart of the patient and the local sense signal indicating the local excitation event at the local position of the heart of the patient. It should be noted that in some embodiments, the control module 106 of the device 100 in FIG. 1 may obtain the local sense signal generated by the discharging of the cardiac muscle cells at the predetermined stimulation position without the pair of stimulation electrodes 103 and 104, while the control module 106 may obtain local sense signal (also referred to as other LS signals) generated by discharging of cardiac muscle cells at other local positions of the heart through other electrodes coupled to the housing and in contact with the heart tissues or blood vessels. The other local positions may be, but are not limited to, adjacent to the predetermined stimulation position. In some other embodiments, the control module 106 may obtain other LS signals from other devices that are communicably connected to the control module 106 and are capable of obtaining electrical signals of cardiac muscle cells at other local positions of the heart of the patient. Later, the control module 106 can determine, based on the GS signal and the LS signal, whether a local excitation event of the cardiac muscle cells at other local positions of the heart of the patient corresponds to a specific global excitation event of the heart of the patient, and transmit, when it is determined that the local excitation event corresponds to the specific global excitation event, an electrical stimulation pulse to the pair of stimulation electrodes during an absolute refractory period of the local excitation event. It should be noted that in some embodiments, "the step of transmitting an electrical stimulation pulse to the pair of stimulation electrodes during an absolute refractory period of the local excitation event" herein can only consider a situation of the absolute refractory period of the local excitation event. In some other embodiments, the above steps can be further limited to considering both the absolute refractory period of the local excitation event and the entire absolute refractory period of the ventricular muscle. This is specifically found in the step and method described in step 604. In some embodiments, the control module 106 may use steps similar to those in the method 600, except that the LS signal is replaced with the other LS signals as described above. In some other embodiments, the control module 106 may adaptively adjust the specific steps and parameters when considering factors such as positional relationships between the predetermined stimulation position and other local positions.

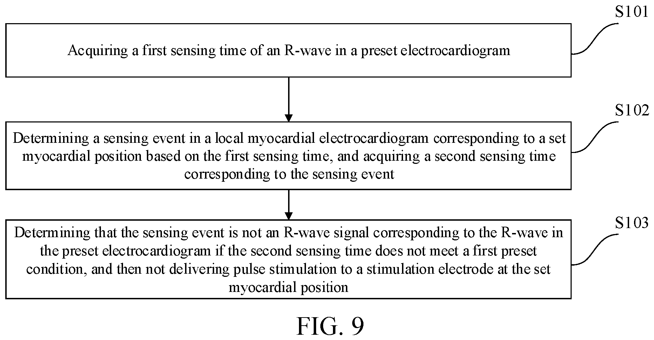

FIG. 9 to FIG. 19 mainly relate to multiple embodiments of the present application, and further describe the specific details of the implantable heart failure treatment device involved in the present application from different aspects. Embodiment 1 and Embodiment 2 relate to a pulse stimulation control method used in the implantable heart failure treatment device of the present application. Embodiment 3 and Embodiment 4 mainly relate to a pulse stimulation control system for an implantable heart failure treatment device. Embodiment 5 mainly relates to a pulse stimulation control device of a pulse stimulation control system. Embodiment 6 relates to an electronic device for implementing a pulse stimulation control method, and Embodiment 7 relates to a computer-readable storage medium having a computer program stored thereon, which is configured to implement the pulse stimulation control method of Embodiment 1 or 2. The specific details of the implantable heart failure treatment device will be further described below in conjunction with FIGS. 9 to 19 and the embodiments described above. It should be noted that these embodiments are only illustrative of the specific implementations of the implantable heart failure treatment device and are not intended to limit the scope of the present application in any way.

**Embodiment 1**

As shown in FIG. 9, the pulse stimulation control method according to this embodiment includes:

S101, acquiring the first sensing time of the R-wave in a preset electrocardiogram,
wherein the preset electrocardiogram includes but is not limited to the surface electrocardiogram (ECG) and the in vivo far-field myocardial electrocardiogram (Far-Field Electrogram, also referred to as FF-EGM); the first sensing time is also referred to as far-field sensing time or GS sensing time;

S102, determining the sensing event in the local myocardial electrocardiogram corresponding to the set myocardial position based on the first sensing time, and acquiring the second sensing time corresponding to the sensing event,

wherein the second sensing time corresponding to the sensing event in the local myocardial electrocardiogram is also referred to as local sensing time or LS sensing time; and

the sensing event in the local myocardial electrocardiogram is a sensing event corresponding to the R-wave in the preset electrocardiogram; and

S103, determining that the sensing event is not the R-wave signal corresponding to the R-wave in the preset electrocardiogram if the second sensing time does not meet the first preset condition, and then not delivering pulse stimulation to the stimulation electrode at the set myocardial position,

wherein the first preset condition is determined based on the first sensing time of the R-wave in the preset electrocardiogram and the second sensing time corresponding to the sensing event.

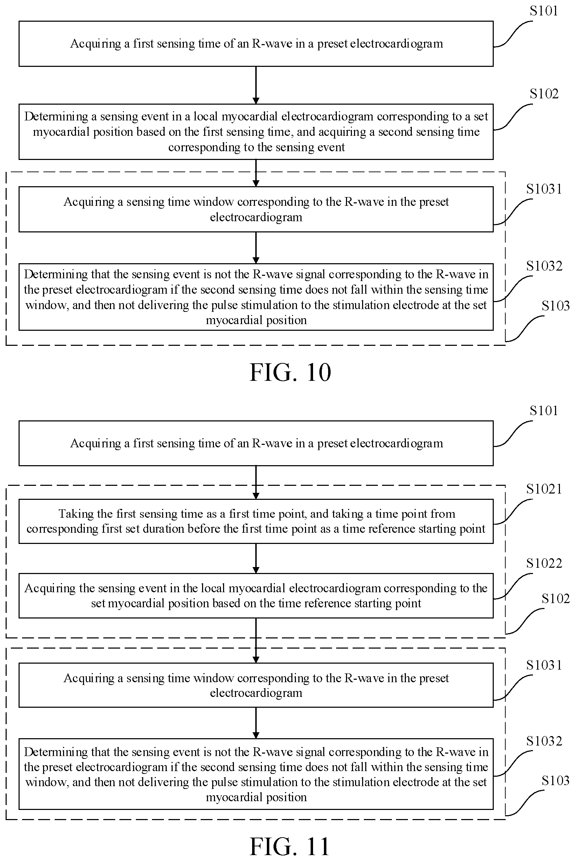

In one implementation, as shown in FIG. 10, step S103 includes:

S1031, acquiring the sensing time window corresponding to the R-wave in the preset electrocardiogram,

wherein the method for acquiring the sensing time window includes but is not limited to: obtaining the sensing time window based on a preset sensing circuit or directly receiving the sensing time window through an external device input.

Surface electrocardiogram (ECG): The corresponding signal is derived from control electrodes attached to the skin; the control electrodes herein include but are not limited to commonly used surface ECG electrodes and specially designed electrodes.

The far-field myocardial electrocardiogram (FF-EGM) represents (or reflects) the signals of the overall cardiac electrical activity, similar to the surface electrocardiogram. The signals corresponding to the far-field myocardial electrocardiogram come from various combinations of control electrodes, including but not limited to electrodes directly contacting the myocardium with a relatively large surface area (i.e., significantly larger than the surface area of conventional electrodes used for electrical stimulation of the myocardium), electrodes not in direct contact with the myocardium but positioned near or distant from the ventricular myocardium, or electrodes with relatively large surface areas located subcutaneously.

For example, different combinations of electrodes correspond to: any combination of different electrodes such as those placed in blood vessels or cardiac chambers, defibrillation electrodes placed on the epicardium, subcutaneous defibrillation electrodes for sub-Q ICDs, or defibrillation electrodes for external defibrillators (such as AEDs). The specific arrangement of these different electrodes may be determined or adjusted based on the requirements of the actual usage scenario.

The sensing time and the corresponding sensing time window may be acquired based on an R-wave in a surface electrocardiogram via a single lead or multiple leads, and/or an R-wave or R-waves in one or more far-field myocardial electrocardiograms. They represent part or all of the time period of overall ventricular myocardial depolarization electrical activity, thereby obtaining the deliverable pulse time window. By improving the control mechanism for determining whether to deliver stimulation at the stimulation site and the timing of delivery, the accuracy of determining pulse stimulation delivery is further enhanced, thereby increasing its safety.

Step S103 further includes S1032: determining that the sensing event is not the R-wave signal corresponding to the R-wave in the preset electrocardiogram if the second sensing time does not fall within the sensing time window, and then not delivering pulse stimulation to the stimulation electrode at the set myocardial position.

Where non-R-wave signals corresponding to the sensing event LS in the local myocardial electrocardiogram include T-wave signals or other interference signals. Specifically, pulse stimulation is CCM stimulation; allowing pulse stimulation to be implemented under different ventricular electrical activities expands the applicability of pulse stimulation, significantly enhancing the overall effectiveness of pulse stimulation control.

CCM stimulation occurs during the following ventricular electrical activities: sinus beats, ventricular beats conducted from atrium, ventricular beats originating from the ventricles (ventricular ectopic excitations), ventricular paced beats, etc.

Confirming that the local sensing event is an R-wave better ensures that CCM stimulation is triggered by the local R-wave instead of other signals (such as T-waves, myoelectricity, or other non-myocardial depolarization electrical activities), effectively eliminating the occurrence of false triggers. Through the design of this critical step, the safety and effectiveness of pulse stimulation can be effectively improved.

To effectively improve the confirmation precision of whether LS in the local myocardial electrocardiogram is R-wave sensing, this embodiment simultaneously considers both far-field sense signal and local sense signal of the same heartbeat. By jointly determining the judgment result through both far-field sense signal and local sense signal under the same heartbeat, the probability of false triggering of pulse stimulation due to potential misjudgment when only considering one type of sensing (either far-field sense signal or local sense signal (LS)) is greatly reduced. Consequently, this significantly enhances the confirmation accuracy of the sensing event and further effectively prevents pulse stimulation from being delivered under incorrect conditions, greatly improving the safety of pulse stimulation for patients.

Ensuring that pulse stimulation is delivered not only during the deliverable period of the local myocardial R-wave (sensed by the electrode at this myocardial site) but also during the deliverable period of the far-field myocardial R-wave (electrical activity generated by the ventricular myocardium as a whole) minimizes and even eliminates the risk of ventricular myocardium depolarization due to inadvertent activation caused by the pulse stimulation.

In this embodiment, the sensing event in the local myocardial electrocardiogram for each heartbeat can be analyzed and processed timely. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, and preventing unnecessary pain or even safety hazards to patients. This ensures patient safety, enhances the reliability of pulse stimulation control, and guarantees the safety, effectiveness, and therapeutic effects of pulse stimulation for patients.

**Embodiment 2**

The pulse stimulation control method according to this embodiment is a further improvement of Embodiment 1, specifically:

In one implementation, as shown in FIG. 11, step S102 includes:

S1021, taking the first sensing time as the first time point, and taking the time point from corresponding first set duration before the first time point as the time reference starting point; and

S1022, acquiring the sensing event in the local myocardial electrocardiogram corresponding to the set myocardial position based on the time reference starting point.

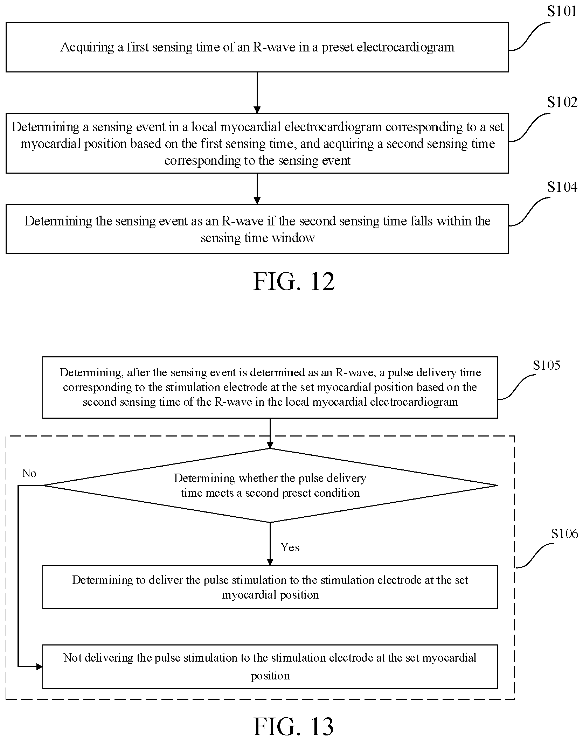

In one implementation, as shown in FIG. 12, step S102 is followed by:

S104, determining the sensing event as an R-wave if the second sensing time falls within the sensing time window. In one implementation, as shown in FIG. 13, the pulse stimulation control method according to this embodiment further includes:

S105, determining, after the sensing event is determined as an R-wave, the pulse delivery time corresponding to the stimulation electrode at the set myocardial position based on the second sensing time of the R-wave in the local myocardial electrocardiogram.

It should be noted that in this embodiment, the method of acquiring sensing events as R-waves may be directly obtained through transmission by external devices, or determined based on judgments such as whether it falls within the sensing time window.

Further included is S106: determining whether the pulse delivery time meets the second preset condition, and if yes, determining to deliver pulse stimulation to the stimulation electrode at the set myocardial position; otherwise, not delivering pulse stimulation to the stimulation electrode at the set myocardial position.

The determination of the sensing event of which the local myocardial sensing time is within the sensing time window corresponding to the R-wave in the preset electrocardiogram ensures that the sensing event belongs to the R-wave signal of the local myocardium corresponding to the R-wave in the preset electrocardiogram. This guarantees the timely delivery of pulse stimulation only when the event is determined as an R-wave, ensuring the timeliness, safety, and effectiveness of pulse stimulation for the patient's heart.

Ensuring that pulse stimulation is delivered not only during the deliverable period of the local myocardial R-wave (sensed by the electrode at this myocardial site) but also during the deliverable period of the far-field myocardial R-wave (electrical activity generated by the ventricular myocardium as a whole) minimizes and even eliminates the risk of the depolarization of other sites of the ventricular myocardium due to the activation of the pulse stimulation. This significantly enhances the safety of pulse stimulation for patients, minimizing unnecessary treatment risks and pain.

In other words, the pulse stimulation control of this embodiment ensures that regardless of where the stimulation electrode is located in the myocardium, pulse stimulation is only delivered during the deliverable period of both the local myocardium and the entire ventricular myocardium under correct conditions. This better ensures the safety and effectiveness of pulse stimulation, allowing pulse stimulation to be delivered under various heartbeats as needed (including ventricular beats caused by atrial conduction, ventricular beats originating from the ventricles themselves, such as PVCs and ventricular paced beats), thereby better meeting the patient's demand for enhanced myocardial contractility.

In one implementation, step S105 includes:

calculating the pulse delivery time corresponding to the stimulation electrode at the set myocardial position based on the second sensing time of the R-wave in the local myocardial electrocardiogram and the preset duration; or,

calculating the time difference between the first sensing time (GS sensing time) corresponding to the R-wave in the preset electrocardiogram and the second sensing time of the set myocardial position; and using the first sensing time as the reference zero point, calculating the pulse delivery time corresponding to the set myocardial position based on the time difference and the preset duration.

The following are the detailed explanations of the processes for calculating the pulse delivery time of the R-wave in the local myocardial electrocardiogram according to the two methods mentioned above:

(1) The second sensing time when the R-wave appears in the local myocardial electrocardiogram is acquired timely, the second sensing time is used as the reference zero point (or called trigger point), and on this basis, the pulse delivery time corresponding to the stimulation electrode at the corresponding myocardial position is obtained by adding the preset duration (LPD).
(2) Based on the second sensing time and the first sensing time when the R-wave appears in the surface electrocardiogram (ECG) and/or the far-field myocardial electrocardiogram (FF-EGM), the time difference (GLSD) between the second sensing time and the first sensing time is calculated; then, using the first sensing time as the reference zero point, and on this basis, the pulse delivery time (GPD) corresponding to each set myocardial position with the first sensing time as the trigger point (reference point) is calculated by adding the time difference (GLSD) and the preset duration (LPD), that is, GPD = GLSD + LPD.

After obtaining the pulse delivery time (GPD) for each set myocardial position, the time difference may be maintained unchanged to deliver cardiac pulse stimulation to the corresponding control electrode based on the pulse delivery time (GPD); there is no need to recalculate before each electrical stimulation output, as the electrical stimulation output time may be directly determined based on GPD after the sensing of the R-wave in the local myocardial electrocardiogram. Directly determining the electrical stimulation output time based on GPD eliminates the need for calculating the pulse stimulation far-field myocardial electrocardiogram delivery time using the first and second sensing times each time, effectively reducing data processing time and improving the efficiency of controlling cardiac pulse stimulation triggers while achieving the cardiac electrical stimulation effects.

In addition, the pulse delivery time (GPD) may be updated regularly or irregularly based on actual needs (where myocardial electrical stimulation may continue or stop). Then, myocardial electrical stimulation continues based on the updated trigger time to achieve more flexible electrical stimulation effects and meet the requirements of various pulse electrical stimulation scenarios.

For pulse stimulation triggered by the R-wave in the local myocardial electrocardiogram (second sensing time), the duration from the R-wave to delivery (LPD) is fixed for each stimulation position (such as a preset duration of 40 ms). For pulse stimulation triggered by the R-wave in the surface electrocardiogram (ECG) and/or far-field myocardial electrocardiogram (FF-EGM), the duration for each stimulation position (GPD) is variable and differs (i.e., determined by a fixed preset duration of 40 ms and a time difference that varies with position). Where the preset duration is typically defaulted to 40 ms, and the duration value may be fine-tuned or updated according to actual needs (i.e., programmable adjustment).

It needs to be noted that the GLSD and GPD corresponding to each set myocardial site can be measured and averaged over several intrinsic heartbeats during the set-up period (defaulting to five consecutive intrinsic heartbeats, ranging from 3 to 12). Alternatively, the GLSD and GPD can be obtained based on the measured duration under each heartbeat. The GPD of each set myocardial site or position is used to trigger the timing of pulse stimulation delivery at that site or position relative to the R-wave sensing moment in the preset electrocardiogram. The pulse delivery time corresponds to the duration from sensing the R-wave to triggering the delivery of cardiac pulse stimulation. The transmission of pulse stimulation at various positions or sites is triggered by the R-wave sensing of surface electrocardiogram (ECG) or the R-wave sensing of far-field myocardial electrocardiogram (FF-EGM), followed by delivering cardiac pulse stimulation to each corresponding control electrode based on the pulse delivery time. To ensure the effectiveness of pulse stimulation, all R-waves (i.e., R-waves in the far-field myocardial electrocardiogram FF-EGM and the surface electrocardiogram ECG) are sensed under the same heartbeat. Based on the aforementioned scheme for acquiring pulse delivery time, the timely and reliable delivery of pulse stimulation can be effectively ensured.

In addition, according to actual needs, GLSD and other parameters compatible with different ventricular electrical activities may be preset, for example, for sinus rhythm (SR) ventricular electrical activity. Then, during actual operation, the corresponding parameters set in the set-up period are directly called upon during the sinus rhythm, reducing the computational requirements for each heartbeat while ensuring the timeliness and effectiveness of pulse stimulation control.

Furthermore, GLSD and other parameters corresponding to the current actual ventricular electrical activity can be dynamically calculated based on real-time cardiac electrical activity sensing data and parameters during the operating period, without relying on parameters such as GLSD obtained during the set-up period. This further improves the timeliness and effectiveness of pulse stimulation control, thereby further ensuring the safety and efficacy of pulse stimulation for patients.

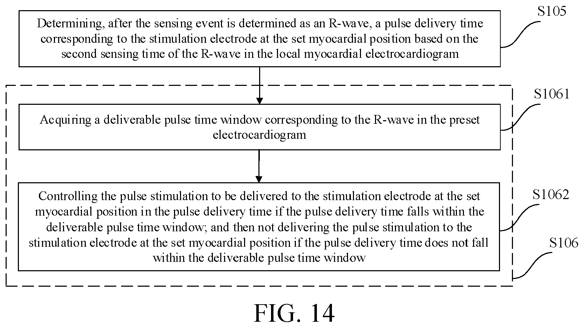

In one implementation, as shown in FIG. 14, step S106 includes:

S1061: acquiring the deliverable pulse time window corresponding to the R-wave in the preset electrocardiogram,

wherein the starting time point and the window duration corresponding to the deliverable pulse time window and the sensing time window can be the same or different; that is, the deliverable pulse time window and the sensing time window may be independently set without mutual correlation or dependency. Specifically, the deliverable pulse time window and the sensing time window are determined based on the preset electrocardiogram, and the specific settings are adaptively adjusted based on factors such as the actual R-wave sensing and myocardial conditions, such that the requirements of the vast majority of pulse stimulation scenarios can be covered or met. Step S106 further includes S1062: controlling the pulse stimulation to be delivered to the stimulation electrode at the set myocardial position in the pulse delivery time if the pulse delivery time falls within the deliverable pulse time window; and

not delivering the pulse stimulation to the stimulation electrode at the set myocardial position if the pulse delivery time does not fall within the deliverable pulse time window.

This scheme ensures that CCM stimulation must occur within the deliverable period of local myocardial depolarization (R-wave) and the deliverable period of in vivo far-field myocardial depolarization (R-wave). Where the deliverable pulse time window corresponds to a safe period, which corresponds to the deliverable period of the entire ventricle. Both the starting point and endpoint of the deliverable pulse time window are adjustable, with the goal of having the starting point approximately correspond to the depolarization zone and having the endpoint occur earlier than or equal to the endpoint of the deliverable period.

In one implementation, when there are a plurality of the set myocardial positions, the pulse stimulation control method according to this embodiment further includes the following steps:

For the sensing event LS in the local myocardial electrocardiogram corresponding to each myocardial position, steps S102 to S106 are executed one by one to timely and effectively deliver pulse stimulation to each myocardial position, ensuring the safety, effectiveness, and reliability of patient treatment.

Where the sensing time window is determined based on the first sensing time of the R-wave in the preset electrocardiogram and the second sensing time corresponding to the sensing event that occurs first.

For example, an illustration is provided by presetting a pulse stimulation electrode at each of three different myocardial positions (A, B, and C) in a patient, the set myocardial positions A, B, and C correspond to stimulation electrode pairs E1, E2, and E3 respectively, and the second sensing times of the corresponding sensing events are LS1, LS2, and LS3 respectively, wherein the occurrence times of LS1, LS2, and LS3 are sequential (i.e., sensing event LS1 occurs first, followed by the other sensing events in subsequent times). Where each electrode pair may be composed of: a unipolar electrode with a unipolar electrode, a bipolar electrode with a bipolar electrode lead, and a combination of a unipolar electrode with a unipolar electrode and a bipolar electrode with a bipolar electrode lead. Additionally, other types of electrodes may be used. The specific types of electrode pairs and how they are combined may be determined or adjusted based on the requirements of the actual scenario.

Specifically, when acquiring, based on the electrode pair E1 at the set myocardium position A, the sensing event LS1 from the local myocardial electrocardiogram corresponding to the myocardial position, the second sensing time corresponding to the sensing event LS1 is acquired, and whether the second sensing time falls within the sensing time window corresponding to the R-wave in the preset electrocardiogram is determined; if the second sensing time does not fall within the window, it is determined that the sensing event LS1 is not the R-wave signal corresponding to the R-wave in the preset electrocardiogram, but rather other interference signals such as T-waves, and thus, the pulse stimulation is prevented from being delivered to the stimulation electrode pair E1 at the set myocardial position A; if the second sensing time falls within the window, it is determined that the sensing event LS1 is the local myocardial R-wave signal corresponding to the R-wave in the preset electrocardiogram, and then the pulse delivery time corresponding to the stimulation electrode corresponding to the set myocardial position A is timely and accurately calculated at the second sensing time corresponding to the sensing event LS1; next, it is determined whether the pulse delivery time falls within the deliverable pulse time window corresponding to the R-wave in the preset electrocardiogram, and if it does, the pulse stimulation is controlled to be delivered to the stimulation electrode at the set myocardial position A at the pulse delivery time, otherwise, it is determined that no pulse stimulation will be delivered to the stimulation electrode at the set myocardial position A, thus completing one pulse stimulation control for the set myocardial position A.

Similarly, the pulse stimulation control process for the set myocardial positions B and C is analogous to the pulse stimulation control process for the set myocardial position A, and therefore, the details are not described herein.

It should be noted that the pulse stimulation control processes for different set myocardial positions are independent of each other and do not cause mutual interference or influence. For example, during the pulse stimulation control process at the set myocardial position A, or after the completed pulse stimulation control at the set myocardial position A, as long as a sensing event LS2 appears in the local myocardial electrocardiogram corresponding to the set myocardial position B, the aforementioned pulse stimulation control process can be independently executed, and the pulse stimulation control for all set myocardial positions will ultimately completed in an orderly and efficient manner, thereby effectively guaranteeing the safety and reliability of pulse stimulation for patients.

In one implementation, when the sensing times corresponding to the sensing events of a plurality of local myocardial electrocardiograms occur very close together (i.e., within a small time span), the pulse stimulation control method according to this embodiment further includes the following steps:

acquiring the second sensing time corresponding to the sensing event LS in the local myocardial electrocardiogram corresponding to each local myocardial electrocardiogram to identify the sensing event which occurs earliest as the first sensing event LS1; acquiring the second sensing time corresponding to the first sensing event LS1, determining whether the first sensing event LS1 falls within the sensing time window corresponding to the R-wave in the preset electrocardiogram, and if the sensing event falls within the window, determining the sensing event as the local myocardial R-wave signal corresponding to the R-wave in the preset electrocardiogram; and

for the sensing events in the local myocardial electrocardiograms corresponding to the remaining myocardial positions, determining whether the second sensing times corresponding to these sensing events fall within the aforementioned sensing time window, and if yes, directly determining that the sensing events in the local myocardial electrocardiograms corresponding to the remaining myocardial positions are also R-wave signals corresponding to the R-wave in the preset electrocardiogram once the first sensing event is determined as the R-wave signal corresponding to the R-wave in the preset electrocardiogram. In this case, there is no need for individual judgment and analysis of the sensing events in the local myocardial electrocardiograms corresponding to the remaining myocardial positions, achieving accurate judgment while greatly simplifying the data analysis process, effectively reducing data processing time, and lowering the computational power requirements of devices. This further ensures the timeliness, accuracy, and effectiveness of the pulse stimulation control for patients. It should be noted that the specific method used to determine whether the sensing events in multiple local myocardial electrocardiograms are R-waves may be selected based on the requirements of the actual scenario. A single execution scheme may be selected or multiple execution schemes may be combined to meet the higher demands of the cardiac electrical ventricular conduction scenario. This greatly enhances the practicality of pulse stimulation control and significantly improves the safety and effectiveness of patient treatment. In addition, when there are a plurality of set myocardial positions, the pulse stimulation control method according to this embodiment further includes:

(1) presetting set sensing parameters corresponding to the R-waves at different set myocardial positions,

wherein the set sensing parameter includes the set sensing time and/or the set sensing occurrence sequence;

(2) setting the sensing events in the other remaining local myocardial electrocardiograms all as R-waves if the sensing event in the local myocardial electrocardiogram that occurs first is an R-wave; or,

(3) setting the sensing events in the other remaining local myocardial electrocardiograms all as R-waves if the sensing event in the local myocardial electrocardiogram that occurs last is an R-wave.

Moreover, the above settings may be adjusted or additional settings may be added based on the requirements of the actual scenario.

The following specifies the implementation principle of determining whether a sensing event in the local myocardial electrocardiogram is an R-wave:

Determining whether the sensing event LS is an R-wave involves two stages: the set-up/pre-set period and the operational period.

For a pulse stimulation system with an electrode for a single myocardial point, this pulse stimulation system will have a preset electrocardiogram (such as ECG) and an electrode lead connected to the myocardium of the ventricle to sense the R-wave, and the system is used to deliver pulse stimulation to the myocardium. During the set-up period, the following parameters need to be measured:

Set-up Period:

(11) ECG Sense (GS sensing time) = The time when the ECG (R-wave) sense occurs (global sense),

wherein the ventricular electrical activity of the R-wave in the preset electrocardiogram (ECG) represents the overall ventricular electrical activity, and R-wave sensing reflects a relatively early time of the ventricular electrical activity.

(12) EGM Sense (LS sensing time) = The time when the EGM (R-wave) sense occurs (local sense), wherein LS is the local electrocardiogram sensing event that sensed first after the time reference starting point corresponding to GS (GS-X, X=60 ms, X can be programmatically controlled within a range of, but not limited to, 30 ms to 200 ms).

(13) GLSD=LS-GS, where GLSD is the time delay between GS and LS.

(14) LPD = The time delay between LS and the pulse stimulation;

(15) GPD = GLSD + LPD, where GPD is the time delay between GS and the pulse stimulation.

(16) GVT is the sensing time window of the corresponding GS (programmable and adjustable); LSVT is the sensing time window of the corresponding LS; specifically, the start time of GVT is GVT-s, and the duration of the time window is B;

a1) GVT-s=GS-A, GLSD>0 (i.e., LS occurs after GS);

GVT-s=GS+GLSD-A, H≤GLSD≤0 (i.e., LS occurs at the same time as or before GS), H<0. Preferably, -120 ms≤H≤ -20 ms.

wherein the default value of A is 20 ms, and A may be programmed and adjusted (including 0 ms); in this case, GVT-s is acquired via the corresponding time parameters of GS and LS.

b1) For sinus rhythm, B ranges from 30 ms to 130 ms (programmable), but is not limited to this range;

c1) For ventricular ectopic excitation, such as PVCs or ventricular paced beats, B ranges from 30 ms to 250 ms (programmable) but is not limited to this range.

LSVT-s=LS-A; LSVT-B=B-(LSVT-s - GVT-s).

It should be noted that LSVT is only used when there are electrodes in a plurality of local myocardial positions; additionally, LS is LS1, i.e., corresponds to the sensing event which occurrs earliest among all myocardial sites.

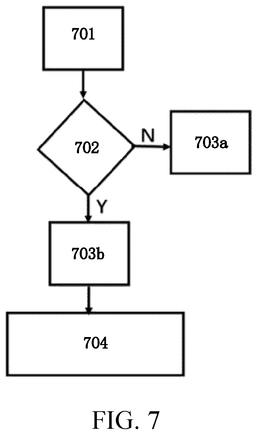

(17) As shown in FIG. 7, GPT is the pulse delivery time window of the corresponding GS; LSPT is the pulse delivery event window of the corresponding LS (programmable and adjustable).

Specifically, the start time of GPT is GPT-s, and GPT-s =GVT-s; the window duration specifically includes, but is not limited to, the range of 20 ms to 200 ms (programmable), with a default of 130 ms.

The start time of LSPT is LSPT-s, and LSPT-s =LSVT-s, and the window duration of LSPT is the window duration of GPT minus GLSD.

It should be noted that the above description includes the scenario where GVT-s corresponds to far-field sense GS in the preset electrocardiogram and LSVT-s corresponds to local sense LS in the local myocardial electrocardiogram (i.e., A = 0 ms).

It should be noted that the above parameters are averaged over several cardiac cycles (e.g., 6 cardiac cycles, which is programmable and may also be a different number of cycles); additionally, the set-up period should be conducted separately during sinus electrical activity, ventricular ectopic activity, ventricular pacing, and the like.

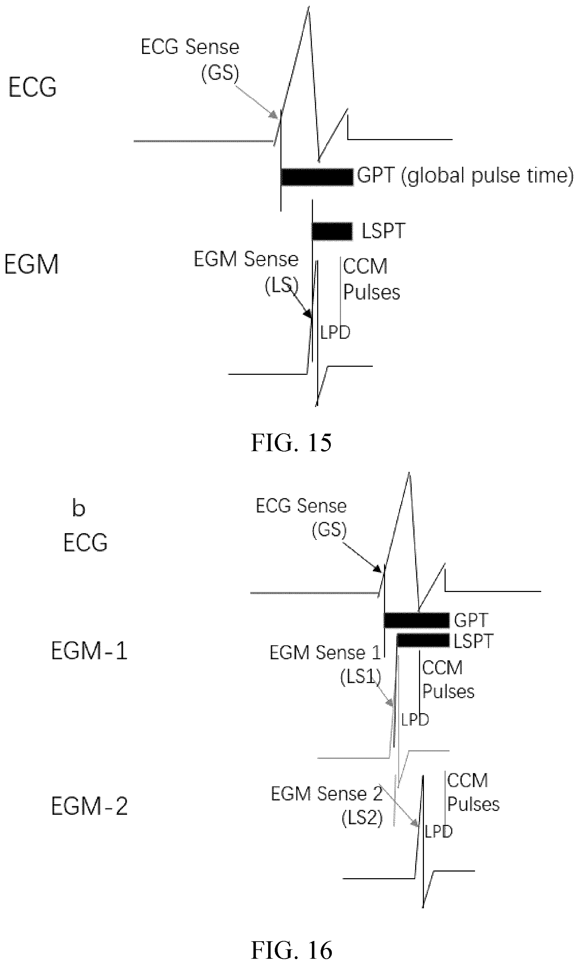

As shown in FIG. 15, for a single electrode lead stimulation system, LSPT is the pulse delivery time window of the corresponding LS, the window starts at LSVT-s, and the window duration is the window duration of GPT (B) minus GLSD. Both serve as the pulse delivery time using LS as the trigger point (reference point) for pulse stimulation delivery.

It should be noted that LSPT is only used when there are electrodes in a plurality of local myocardial positions; additionally, LS is LS1, i.e., corresponds to the sensing event which occurs earliest among all myocardial sites. In this case, the starting point of GPT also is acquired via the corresponding time parameters of LS.

In addition, in this embodiment, the time windows such as GVT, GPT, LSVT, and LSPT are all described by two parameters, i.e., the corresponding window starting point and the window duration.

Operational Period:

(18) LS as a real-time local R-wave sensing needs to meet the following conditions:

a2) There are two sensing events: the preset ECG (GS) and the local EGM (LS), wherein LS is the second sensing time of the local electrocardiogram sensing event that is sensed first after the time reference starting point corresponding to GS (GS-X, X=60 ms, X can be programmatically controlled within a range of, but not limited to, 30 ms to 200 ms);

b2) LS falls within GVT (i.e., the sensing time window).

(19) The criteria for determining whether the pulse stimulation time is appropriate:

a3) The pulse stimulation time falls within GPT (the pulse delivery time window). Where GVT and GPT are derived from the corresponding ventricular electrical activities acquired during the set-up period, such as sinus electrical activity, ventricular ectopic activity, or ventricular pacing.

For a pulse stimulation system with a plurality of electrodes, this pulse stimulation system will have a preset electrocardiogram and a plurality of leads connected to multiple myocardial sites of the ventricles to sense the R-wave, and the system is used to deliver pulse stimulation to the local myocardium. During the set-up period, the following parameters need to be measured:

Set-up Period:

(21) Similar to the above (11) to (19), measure and acquire all parameters corresponding to each stimulation electrode (as some programmatically controlled numbers together with the measured numbers constitute all parameters).

(22) Pre-form templates corresponding to LSn (local R-wave sensing), which include the time of LSn (n=1, 2, 3, ...) relative to LS1, and information such as the sequence of the occurrence of each electrode sensing.

Operational Period:

(23) Acquire GS and LSn in real time during the operation.

(24) Apply the rules from step (18) above to determine whether each LSn is a true R-wave.

Alternatively, if LS1 is confirmed as a true R-wave in step (18), then LSn (n=2, 3, ...) is assumed to be a true R-wave (if LSn occurs within the GVT window).

Alternatively, if LS1 is confirmed as a true R-wave in step (18) and LSn (n=2, 3, ...) conforms to the preset template, then LSn (n=2, 3, ...) is separately confirmed as a true R sensing.

Alternatively, if LS1 is used as a reference, the sensing time window will be LSVT, and it is determined whether LSn (n=2, 3, ...) is within the LSVT time window.

As shown in FIG. 16, for a multi-electrode lead stimulation system during the setup period: if LS1 is used as a reference, the pulse delivery time is LSPT.

Operational period: after confirming each LS as R-wave sensing, the corresponding pulse delivery time should be within the GPT window; if LS1 is used as the trigger point (reference point) for the pulse delivery time, then the delivery time of LSn (n>1) is within the LSPT window.

It should be noted that for single-electrode and multi-electrode pulse stimulation systems, the pulse stimulation delivery time relative to each myocardial position is after the local myocardial sensing time, for example, after 40 ms. GPT or LSPT represents new requirements for pulse stimulation delivery time, especially in cases where there is pulse stimulation delivery for multiple stimulation sites. The delivery time of each stimulation site needs to fall within the time window of GPT or LSPT, that is, within the deliverable period of the entire ventricle of the same heartbeat (ventricular excitation), not just the deliverable period of local ventricular muscle at the electrode site.

GVT/LSVT, as a "global ventricular" or "far-field ventricular" R-wave sensing time window, is used to determine if LS is R-wave sensing of local ventricular depolarization corresponding to GS (i.e., corresponding to the "global ventricular" or "far-field ventricular" R-wave sensing of ventricular depolarization). GPT/LSPT, as the pulse deliverable time window (serving as a safety zone for stimulation delivery, corresponding to the deliverable period of the ventricular muscle as a whole ("global ventricle" or "far-field ventricle")), is used to determine if the pulse stimulation delivery time corresponding to LS is safe. GPT and GVT are independent parameters that can be separately controlled by a program to meet actual parameter configuration requirements. Moreover, to reduce the complexity of programming control, doctors may select the same numerical values for both parameters (when appropriate). Alternatively, the system may directly assign the same numerical value to both parameters in advance, but the functionality of programmatically controlling the two parameters separately must be maintained. The two steps may be used together or separately.

In addition, besides the aforementioned method of first setting a set-up period and then performing an operational period (i.e., the operational period relies on the numbers obtained during the set-up period for specific execution), the parameters may also be directly obtained and used during each electrical activity of ventricular muscle (each cardiac cycle) in the operational period, without relying on the set-up period. This greatly enhances the flexibility and efficiency of the pulse stimulation control process.

In this embodiment, pulse stimulation is delivered only after the R-wave, allowing for timely analysis and processing of the sensing event in the local myocardial electrocardiogram of the myocardium. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, thereby preventing unnecessary pain or even safety hazards to patients. This ensures patient safety and enhances the reliability of pulse stimulation control; additionally, it ensures that pulse stimulation is delivered timely when the sensing event is confirmed as an R-wave, that is, pulse stimulation is delivered only under correct conditions.

In addition, pulse stimulation is only delivered during the ventricular deliverable period: By confirming that the pulse stimulation delivery time falls within the pulse delivery window corresponding to the overall ventricular electrical activity (R-wave) in the surface electrocardiogram or the in vivo far-field myocardial electrocardiogram, the timeliness, safety, and effectiveness of pulse stimulation to the patient's heart are guaranteed. Where for the safety of the pulse stimulation delivery time, using the surface electrocardiogram or the far-field myocardial electrocardiogram to obtain the deliverable period information of ventricular electrical activity also represents a positive improvement in pulse stimulation technology, further ensuring the safety, effectiveness, and therapeutic effect of pulse stimulation for the patient.

## Embodiment 3

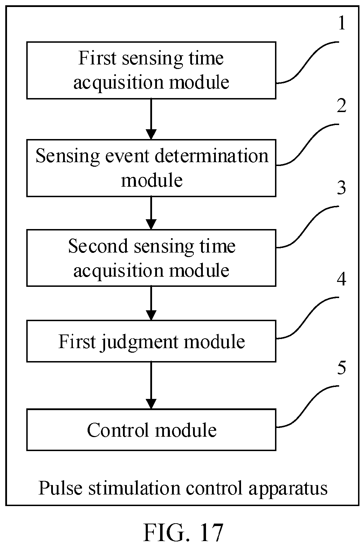

As shown in FIG. 17, the pulse stimulation control apparatus according to this embodiment includes:

a first sensing time acquisition module 1, configured to acquire the first sensing time of the R-wave in a preset electrocardiogram, wherein the preset electrocardiogram includes but is not limited to the surface electrocardiogram (ECG) and the in vivo far-field myocardial electrocardiogram (Far-Field Electrogram); the first sensing time is also referred to as GS;

a sensing event determination module 2, configured to determine the sensing event in the local myocardial electrocardiogram corresponding to the set myocardial position based on the first sensing time;

a second sensing time acquisition module 3, configured to acquire the second sensing time corresponding to the sensing event,

wherein the second sensing time corresponding to the sensing event in the local myocardial electrocardiogram is also referred to as LS;

the sensing event in the local myocardial electrocardiogram is a sensing event corresponding to the R-wave in the preset electrocardiogram;

a first judgment module 4, configured to determine that the sensing event is not the R-wave signal corresponding to the R-wave in the preset electrocardiogram if the second sensing time does not meet the first preset condition and to call the control module 5; and

a control module 5, configured to not deliver the pulse stimulation to the stimulation electrode at the set myocardial position,

wherein the first preset condition is determined based on the first sensing time of the R-wave in the preset electrocardiogram and the second sensing time corresponding to the sensing event.

In one implementation, the first judgment module 4 is further configured to acquire the sensing time window corresponding to the R-wave in the preset electrocardiogram; the first judgment module 4 is further configured to determine that the sensing event is not the R-wave signal corresponding to the R-wave in the preset electrocardiogram if the second sensing time does not fall within the sensing time window.

Where the method for acquiring the sensing time window includes but is not limited to: obtaining the sensing time window based on a preset sensing circuit or directly receiving the sensing time window through an external device input.

Non-R-wave signals corresponding to the sensing event LS in the local myocardial electrocardiogram include T-wave signals or other interference signals. Specifically, pulse stimulation is CCM stimulation; allowing pulse stimulation to be implemented under different ventricular electrical activities expands the applicability of pulse stimulation, significantly enhancing the overall effectiveness of pulse stimulation control.

CCM stimulation occurs during the following ventricular electrical activities: sinus beats, ventricular beats resulting from atrial conduction, ventricular beats originating from the ventricles (ventricular ectopic excitations), ventricular paced beats, etc.

Confirming that the local sensing event is an R-wave better ensures that CCM stimulation is triggered by the local R-wave instead of other signals (such as T-waves, myoelectricity, or other non-myocardial depolarization electrical activities), effectively eliminating the occurrence of false triggers. Through the design of this critical step, the safety and effectiveness of pulse stimulation can be effectively improved.

It should be noted that the implementation principle of the pulse stimulation control apparatus in this embodiment is similar to that of the pulse stimulation control method in Embodiment 1, and therefore, the details are not described herein.

In this embodiment, the sensing event in the local myocardial electrocardiogram for each heartbeat can be analyzed and processed timely. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, and preventing unnecessary pain or even safety hazards to patients. This ensures patient safety, enhances the reliability of pulse stimulation control, and guarantees the safety, effectiveness, and therapeutic effects of pulse stimulation for patients.

**Embodiment 4**

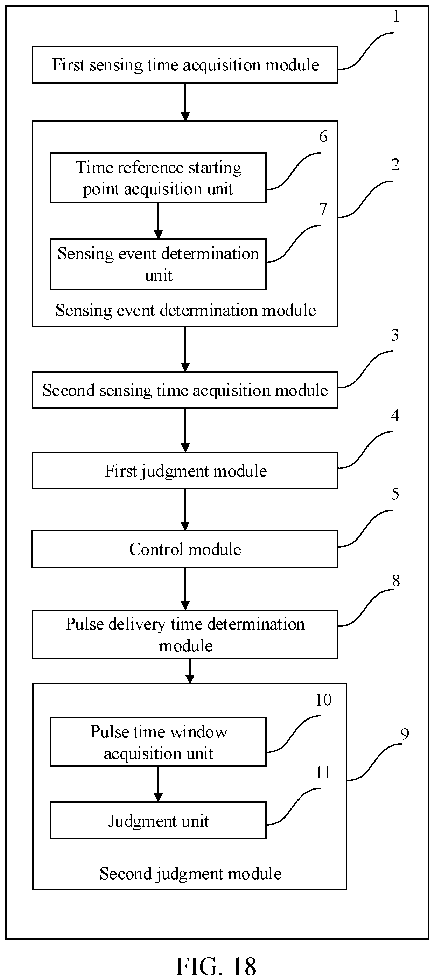

As shown in FIG. 18, the pulse stimulation control system according to this embodiment is a further improvement of Embodiment 3, specifically:

In one implementation, the sensing event determination module 2 according to this embodiment includes:

a time reference starting point acquisition unit 6, configured to take the first sensing time as the first time point and take the time point from corresponding first set duration before the first time point as the time reference starting point; and

a sensing event determination unit 7, configured to acquire the sensing event in the local myocardial electrocardiogram corresponding to the set myocardial position based on the time reference starting point.

In one implementation, the first judgment module 4 is further configured to determine the sensing event as an R-wave if the second sensing time falls within the sensing time window.

In one implementation, the pulse stimulation control system according to this embodiment further includes:

a pulse delivery time determination module 8, configured to determine, after the sensing event is determined as an R-wave, the pulse delivery time corresponding to the stimulation electrode at the set myocardial position based on the second sensing time of the R-wave in the local myocardial electrocardiogram.

It should be noted that in this embodiment, the method of acquiring sensing events as R-waves may be directly obtained through transmission by external devices, or determined based on judgments such as whether it falls within the sensing time window. Specifically, the pulse delivery time determination module 8 is configured to calculate the pulse delivery time corresponding to the stimulation electrode at the set myocardial position based on the second sensing time of the R-wave in the local myocardial electrocardiogram and the preset duration.

Alternatively, the pulse delivery time determination module 8 is configured to acquire the first sensing time corresponding to the R-wave in the preset electrocardiogram; calculate the time difference between the first sensing time and the second sensing time at the set myocardial position; and calculate, using the first sensing time as the reference zero point, the pulse delivery time corresponding to the set myocardial position based on the time difference and the preset duration.

A second judgment module 9 is configured to determine whether the pulse delivery time meets the second preset condition; if yes, the judging module is called to determine to deliver pulse stimulation to the stimulation electrode at the set myocardial position; otherwise, the judging module is called to not deliver pulse stimulation to the stimulation electrode at the set myocardial position.

The determination of the sensing event of which the local myocardial sensing time is within the sensing time window corresponding to the R-wave in the preset electrocardiogram ensures that the sensing event belongs to the R-wave signal of the local myocardium corresponding to the R-wave in the preset electrocardiogram. This guarantees the timely delivery of pulse stimulation only when the event is determined as an R-wave, ensuring the timeliness, safety, and effectiveness of pulse stimulation for the patient's heart.

In one implementation, the second judgment module 9 according to this embodiment includes:

a pulse time window acquisition unit 10, configured to acquire the deliverable pulse time window corresponding to the R-wave in the preset electrocardiogram, wherein the deliverable pulse time window is set to correspond with the sensing time window; and a judgment unit 11, configured to call the control module 5 to control the pulse stimulation to be delivered to the stimulation electrode at the set myocardial position in the pulse delivery time if the pulse delivery time falls within the deliverable pulse time window, and further configured to call the control module 5 to not deliver the pulse stimulation to the stimulation electrode at the set myocardial position if the pulse delivery time does not fall within the deliverable pulse time window.

In one implementation, when there are a plurality of the set myocardial positions, the control module 5 in this embodiment is configured to control the first judgment module 4 to determine whether the corresponding second sensing time falls within the sensing time window for each of the second sensing times of the sensing events occurring in each of the local myocardial electrocardiograms in sequence. Where the sensing time window is determined based on the first sensing time of the R-wave in the preset electrocardiogram and the second sensing time corresponding to the sensing event that occurs first.

In one implementation, when there are a plurality of the set myocardial positions, the control module 5 in this embodiment is configured to preset set sensing parameters corresponding to the R-wave at different set myocardial positions,

wherein the set sensing parameter includes the set sensing time and/or the set sensing occurrence sequence;
or,
set the sensing events in the other remaining local myocardial electrocardiograms all as R-waves if the sensing event in the local myocardial electrocardiogram that occurs first is an R-wave;
or,
set the sensing events in the other remaining local myocardial electrocardiograms all as R-waves if the sensing event in the local myocardial electrocardiogram that occurs last is an R-wave.

It should be noted that the working principle of the pulse stimulation control apparatus in this embodiment is similar to the implementation principle of pulse stimulation control in Embodiment 2, and therefore, the details are not described herein.

In this embodiment, pulse stimulation is delivered only after the R-wave, allowing for timely analysis and processing of the sensing event in the local myocardial electrocardiogram of the myocardium. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, thereby preventing unnecessary pain or even safety hazards to patients. This ensures patient safety and enhances the reliability of pulse stimulation control; additionally, it ensures that pulse stimulation is delivered timely when the sensing event is confirmed as an R-wave, that is, pulse stimulation is delivered only under correct conditions.

In addition, pulse stimulation is only delivered during the ventricular deliverable period: By confirming that the pulse stimulation delivery time falls within the pulse delivery window corresponding to the overall ventricular electrical activity (R-wave) in the surface electrocardiogram or the in vivo far-field myocardial electrocardiogram, the timeliness, safety, and effectiveness of pulse stimulation to the patient's heart are guaranteed. Where for the safety of the pulse stimulation transmission time, using the surface electrocardiogram or the far-field myocardial electrocardiogram to obtain the deliverable period information of ventricular electrical activity also represents a positive improvement in pulse stimulation technology, further ensuring the safety, effectiveness, and therapeutic effect of pulse stimulation for the patient.

## Embodiment 5

The medical system ECS in this embodiment includes the pulse stimulation control apparatus in Embodiments 3 or 4.

Global myocardial depolarization (R-wave and corresponding GS) may originate from, but is not limited to, a surface electrocardiogram (from surface electrocardiogram electrodes attached to the skin), or a far-field myocardial electrocardiogram (FF-EGM, from electrodes near the heart or electrodes distant from the heart but located subcutaneously within the body in tissues or organs). This allows the ECS system to be used in various devices or equipment that provide cardiac therapy, such as AEDs, MCS (Mechanical Circulatory Support), subQ-ICDs, and implantable CRM (Cardiac Rhythm Management) devices, among others. Additionally, this enables the ECS to serve not only as an acute support device (with surface electrocardiogram ECG as one of the input signals) but also as a chronic heart failure treatment device, although surface ECG cannot always be used as an input for the device. In this way, the ECS system can provide treatment to more patients in more scenarios, thereby benefiting more patients. The medical system in this embodiment allows pulse stimulation to be delivered during sinus rhythm, ventricular rhythm (beats) conducted from the atrium, or other ventricular ectopic rhythms (beats). This represents a breakthrough or improvement over the current limitation where pulse stimulation cannot be delivered during heartbeats under a non-normal cardiac conduction system, such as sinus beats, ventricular beats resulting from atrial conduction, ventricular beats originating from the ventricles (ventricular ectopic excitations), and ventricular paced beats. This will provide more opportunities for cardiac support, especially in situations where the patient may need such support the most.

In this embodiment, the medical system integrates the aforementioned pulse stimulation control apparatus, which can ensure that pulse stimulation is only delivered under correct conditions and not under incorrect conditions. Moreover, the medical system delivers pulse stimulation within the deliverable period corresponding to the R-wave in surface electrocardiograms or in vivo far-field myocardial electrocardiograms, ensuring the timeliness and effectiveness of the pulse stimulation to the patient's heart. This guarantees the safety, effectiveness, and therapeutic efficacy of the pulse stimulation for the patient, thereby effectively enhancing the overall product performance of existing medical systems.

## Embodiment 6

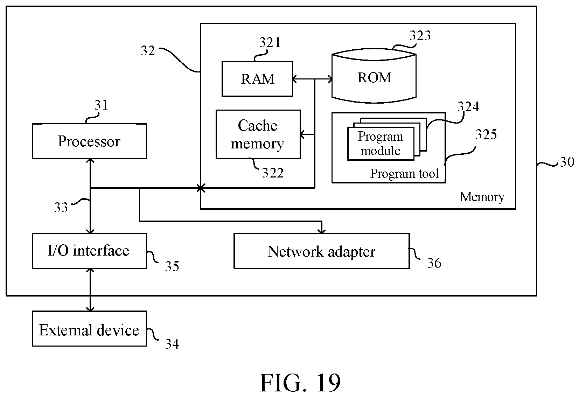

FIG. 19 is a structural schematic diagram of an electronic device provided according to Embodiment 6 of the present disclosure. The electronic device includes a memory, a processor, and a computer program stored on the memory and executable on the processor. The processor, when executing the program, implements the pulse stimulation control method according to Embodiment 1 or 2. The electronic device 30 shown in FIG. 19 is only an example, and should not bring any limitation to the functions and the use scope of the embodiments of the present disclosure.

As shown in FIG. 19, the electronic device 30 may take the form of a general-purpose computing device, such as a server device. Components of the electronic device 30 may include, but are not limited to: at least one processor 31, at least one memory 32, and a bus 33 connecting various system components (including the memory 32 and the processor 31). The bus 33 includes a data bus, an address bus, and a control bus. The memory 32 may include a volatile memory, such as a random access memory (RAM) 321 and/or a cache memory 322, and may further include a read-only memory (ROM) 323. The memory 32 may further include programs/utilities 325 with a set of (at least one) program modules 324, such program modules 324 including but not limited to: an operating system, one or more application programs, other program modules, and program data. Each of these examples or some combination thereof may include the implementation of a network environment. The processor 31 executes various functional applications and performs data processing, such as the pulse stimulation control method according to Embodiment 1 or 2 of the present disclosure, by executing the computer program stored in the memory 32. The electronic device 30 may also communicate with one or more external devices 34 (such as a keyboard and a pointing device). Such communication may be implemented through an input/output (I/O) interface 35. In addition, a model-generating device 30 may also communicate with one or more networks (such as a local area network (LAN), a wide area network (WAN), and/or a public network, such as the Internet) via a network adapter 36.

As shown in FIG. 19, the network adapter 36 communicates with other modules of the model-generating device 30 via the bus 33. It should be understood that although not shown in the figures, other hardware and/or software modules may be used in conjunction with the model-generating device 30, including, but not limited to: microcode, device drivers, redundant processors, external disk drive arrays, RAID (disk array) systems, tape drives, data backup storage systems, and the like.

It should be noted that although several units/modules or sub-units/modules of the electronic device are mentioned in the above detailed description, such a division is merely exemplary but not mandatory. Indeed, the features and functions of two or more of the units/modules described above may be embodied in one unit/module according to embodiments of the present disclosure. Conversely, the features and functions of one unit/module described above may be further divided to be embodied by a plurality of units/modules.

## Embodiment 7

This embodiment provides a computer-readable storage medium storing a computer program. The program, when executed by a processor, causes the processor to implement the steps of the pulse stimulation control method in Embodiment 1 or 2.

Where the readable storage medium may be employed more specifically and may include, but is not limited to: a portable disk, a hard disk, a random access memory, a read-only memory, an erasable programmable read-only memory, an optical storage device, a magnetic storage device, or any suitable combination of the foregoing.

In a possible implementation, the present disclosure can also be implemented in the form of a program product comprising program codes used for causing a terminal device to implement the steps of the pulse stimulation control method in Embodiment 1 or 2 when the program product is executed on the terminal device.

Where the program codes used for implementing the present disclosure are written in any combination of one or more programming languages, and the program codes may be executed entirely on a user device, partly on the user device, as a stand-alone software package, partly on the user device and partly on a remote device, or entirely on the remote device.

It should be noted that although the above detailed description explains the implantable heart failure treatment device and several steps or modules for heart failure treatment based on the device, this division is only exemplary and not mandatory. In fact, according to the embodiments of the present application, the features and functions of the two or more modules described above can be embodied in one module. On the contrary, the features and functions of one module described above can be embodied by multiple modules.

A person skilled in the art can understand and implement other changes to the disclosed implementations by studying this specification, the disclosed content, the accompanying drawings, and the attached claims. In the claims, the term "comprise" does not exclude other elements and steps, and the term "a/an" and "one" do not exclude the plural. In the practical application of the present application, a part may perform the functions of multiple technical features referenced in the claims. Any reference numerals in the claims should not be construed as limiting the scope.

## Claims

1. An implantable heart failure treatment device, wherein the device comprises:

   a housing;
   an anchoring member connected to the housing and configured to fix the housing to a heart of a patient;
   a pair of stimulation electrodes coupled to the housing, configured to be in contact with a predetermined stimulation position of the heart of the patient, and configured to apply an electrical stimulation pulse for enhancing cardiomyocyte contractility of the heart of the patient to the predetermined stimulation position;
   a pulse generation module accommodated within the housing, electrically coupled to the pair of stimulation electrodes, and configured to generate the electrical stimulation pulse; and
   a control module accommodated within the housing, electrically coupled to the pair of stimulation electrodes and the pulse generation module, and configured to receive a far-field sense signal indicating a global excitation event of the heart of the patient and a local sense signal indicating a local excitation event of a local position of the heart of the patient; and determine, at least based on the far-field sense signal and the local sense signal, whether the local excitation event of the local position of the heart of the patient corresponds to a specific global excitation event, and transmit, when it is determined that the local excitation event corresponds to the specific global excitation event, the electrical stimulation pulse to the pair of stimulation electrodes during an absolute refractory period of the local excitation event.

2. The implantable heart failure treatment device according to claim 1, wherein the pair of stimulation electrodes are configured to sense a local sense signal generated by discharging of cardiac muscle cells at the predetermined stimulation position, and the local sense signal indicating the local excitation event of the local position of the heart of the patient comprises the local sense signal sensed by the pair of stimulation electrodes and generated by the discharging of the cardiac muscle cells at the predetermined stimulation position.

3. The implantable heart failure treatment device according to claim 1, wherein the housing is configured to be suitable for being accommodated within a cardiac chamber of the heart of the patient, and the predetermined stimulation position comprises an endocardium.

4. The implantable heart failure treatment device according to claim 1, wherein the housing is configured to be suitable for being accommodated within a vein on an outer-side surface of the heart of the patient, and the predetermined stimulation position comprises an epicardium.

5. The implantable heart failure treatment device according to claim 1, wherein the device further comprises a far-field excitation sense module configured to sense the global excitation event of the heart of the patient and provide the far-field sense signal indicating the global excitation event to the control module.

6. The implantable heart failure treatment device according to claim 5, wherein the far-field excitation sense module comprises a pair of sensing electrodes, the pair of sensing electrodes is coupled to the housing and is configured to sense the far-field sense signal indicating the global excitation event of the heart of the patient and provide the far-field sense signal to the control module.

7. The implantable heart failure treatment device according to claim 6, wherein a surface area of the sensing electrode is larger than a surface area of the stimulation electrode.

8. The implantable heart failure treatment device according to claim 6, wherein the pair of sensing electrodes and the pair of stimulation electrodes share one electrode, and the common electrode serves as a reference potential electrode.

9. The implantable heart failure treatment device according to claim 1, wherein determining, performed by the control module and based at least on the far-field sense signal and the local sense signal, whether the local excitation event of the local position of the heart of the patient corresponds to a specific global excitation event comprises:

obtaining a specific event time window corresponding to the specific global excitation event; and
if a time of sensing the local excitation event falls into the specific event time window, determining that the local excitation event corresponds to the specific global excitation event.

10. The implantable heart failure treatment device according to claim 1, wherein transmitting the electrical stimulation pulse to the pair of stimulation electrodes during an absolute refractory period of the local excitation event, comprises:

determining a pulse delivery time based on the local sense signal and/or the far-field sense signal;
determining, based on the local sense signal and/or the far-field sense signal, a pulse deliverable time window suitable for transmitting the electrical stimulation pulse; and
when the pulse delivery time falls into the pulse deliverable time window, transmitting the electrical stimulation pulse to the pair of stimulation electrodes.

11. The implantable heart failure treatment device according to claim 1, wherein the specific global excitation event corresponds to an R wave or a QRS wave complex in a far-field electrocardiogram, and the local excitation event corresponds to an R wave or a QRS wave complex in a local myocardial electrocardiogram.

12. The implantable heart failure treatment device according to claim 1, wherein the control module is further configured to: determine an electrical signal source causing the local excitation event; and determine, at least based on the electrical signal source, the local sense signal, and the far-field sense signal, whether the local excitation event of the cardiac muscle cells at the predetermined stimulation position corresponds to the specific global excitation event.

13. The implantable heart failure treatment device according to claim 12, wherein determining, at least based on the electrical signal source, the local sense signal, and the far-field sense signal, whether the local excitation event of the cardiac muscle cells at the predetermined stimulation position corresponds to the specific global excitation event comprises: determining a length of a specific event time window according to the electrical signal source.

14. The implantable heart failure treatment device according to claim 12, wherein transmitting the electrical stimulation pulse to the pair of stimulation electrodes during an absolute refractory period of the local excitation event comprises:

> determining, based on the electrical signal source and further in conjunction with the local sense signal and/or the far-field sense signal, a pulse delivery time;
> determining, based on the electrical signal source and further in conjunction with the local sense signal and/or the far-field sense signal, a pulse deliverable time window suitable for transmitting the electrical stimulation pulse; and
> when the pulse delivery time falls into the pulse deliverable time window, transmitting the electrical stimulation pulse to the pair of stimulation electrodes.

15. The implantable heart failure treatment device according to claim 12, wherein the electrical signal source causing the local excitation event comprises a self-generated impulse from an atrial position, a self-generated impulse from a ventricular position, or an external electrical stimulation applied to the heart.

16. The implantable heart failure treatment device according to claim 6, wherein at least one of the pair of stimulation electrodes is placed in the anchoring member, and the pair of sensing electrodes is placed within the housing and is exposed out of a surface of the housing.

17. The implantable heart failure treatment device according to claim 6, wherein the far-field excitation sense module is disposed outside the housing and is communicably coupled to the control module.

18. The implantable heart failure treatment device according to claim 1, wherein the far-field sense signal received by the control module and indicating the global excitation event of the heart of the patient is from another implantable device communicably connected to the control module or is from a non-implantable device communicably connected to the control module.

19. The implantable heart failure treatment device according to claim 1, wherein the device further comprises another pair or multiple pairs of stimulation electrodes; the another pair or multiple pairs of stimulation electrodes are coupled to the housing and are configured to be in contact with one or more other predetermined stimulation positions of the heart of the patient; each of the another pair or multiple pairs of stimulation electrodes is configured to respectively sense a local sense signal generated by discharging of cardiac muscle cells at another predetermined stimulation position and apply an electrical stimulation pulse to the another predetermined stimulation position.

20. The implantable heart failure treatment device according to claim 1, wherein the pair of stimulation electrodes is further configured to selectively apply an electrical pacing pulse for adjusting a heart rate of the heart of the patient to the predetermined stimulation position, and the control module is further configured to determine, at least based on the far-field sense signal and the electrical pacing pulse, whether the local excitation event of the cardiac muscle cells at the predetermined stimulation position corresponds to the specific global excitation event.

21. The implantable heart failure treatment device according to claim 20, wherein the control module is further configured to receive a model selection signal, and control, according to the mode selection signal, the implantable heart failure treatment device to be in a pacemaker mode or a non-pacemaker mode, wherein in the non-pacemaker mode, the control module determines, at least based on the far-field sense signal and the local sense signal, whether the local excitation event of the cardiac muscle cells at the predetermined stimulation position corresponds to the specific global excitation event; and in the pacemaker mode, the control module determines, at least based on the far-field sense signal and the electrical pacing pulse, whether the local excitation event of the cardiac muscle cells at the predetermined stimulation position corresponds to the specific global excitation event.

22. The implantable heart failure treatment device according to claim 21, wherein in the pacemaker mode, the control module is configured to determine capture of the heart of the patient, and to determine a start point of the local excitation event of the cardiac muscle cells at the predetermined stimulation position based on an applying time of the electrical pacing pulse after the capture is established.

23. The implantable heart failure treatment device according to claim 22, wherein the control module is configured to determine the capture of the heart of the patient based on the far-field sense signal.

24. The implantable heart failure treatment device according to claim 1, wherein the device is a leadless device, and the device further comprises:

> a first power supply and a second power supply which are accommodated within the housing, wherein the first power supply is configured to supply power to the pulse generation module and the control module; and
> a pacing pulse generation module accommodated within the housing, electrically coupled to the second power supply, powered by the second power supply, and configured to generate the electrical pacing pulse.

25. An implantable heart failure treatment device, wherein the device comprises:

a housing;

an anchoring member connected to the housing and configured to fix the housing to a heart of a patient;

a pair of stimulation electrodes coupled to the housing, configured to be in contact with a predetermined stimulation position of the heart of the patient, and configured to apply to the predetermined stimulation position an electrical stimulation pulse for enhancing cardiomyocyte contractility of the heart of the patient, and an electrical pacing pulse for adjusting a heart rate of the heart of the patient;

a pulse generation module accommodated within the housing, electrically coupled to the pair of stimulation electrodes, and configured to generate the electrical stimulation pulse and the electrical pacing pulse; and

a control module accommodated within the housing, electrically coupled to the pair of stimulation electrodes and the pulse generation module, and configured to: receive a far-field sense signal indicating a global excitation event of the heart of the patient; determine, at least based on the far-field sense signal and the applied electrical pacing pulse, whether a local excitation event of cardiac muscle cells at the predetermined stimulation position corresponds to a specific global excitation event; and transmit, when it is determined that the local excitation event corresponds to the specific global excitation event, the electrical stimulation pulse to the pair of stimulation electrodes during an absolute refractory period of the local excitation event.

26. The implantable heart failure treatment device according to claim 25, wherein determining, at least based on the far-field sense signal and the applied electrical pacing pulse, whether a local excitation event of cardiac muscle cells at the predetermined stimulation position corresponds to a specific global excitation event comprises:

obtaining a specific event time window corresponding to the specific global excitation event; and

if an applying time of the electrical pacing pulse falls into the specific event time window, determining that the local excitation event corresponds to the specific global excitation event.

27. The implantable heart failure treatment device according to claim 25, wherein determining, at least based on the far-field sense signal and the applied electrical pacing pulse, whether a local excitation event of cardiac muscle cells at the predetermined stimulation position corresponds to a specific global excitation event comprises:

after the pair of stimulation electrodes applies the electrical pacing pulse for adjusting the heart rate of the heart of the patient to the predetermined stimulation position of the heart of the patient, sensing capture of the heart of the patient; and

after the capture of the heart of the patient has been sensed, determining that the local excitation event of the cardiac muscle cells at the predetermined stimulation position corresponds to the specific global excitation event.

28. An implantable heart failure treatment device, wherein the device comprises:

a housing;

an anchoring member connected to the housing and configured to fix the housing to a heart of a patient;

a pair of stimulation electrodes coupled to the housing, configured to be in contact with a predetermined stimulation position of the heart of the patient, and configured to sense a local sense signal generated by discharging of cardiac muscle cells at the predetermined stimulation position and apply an electrical stimulation pulse for enhancing cardiomyocyte contractility of the heart of the patient to the predetermined stimulation position;

a pulse generation module accommodated within the housing, electrically coupled to the pair of stimulation electrodes, and configured to generate the electrical stimulation pulse; and

a control module accommodated within the housing, electrically coupled to the pair of stimulation electrodes and the pulse generation module, and configured to: receive a far-field sense signal indicating a global excitation event of the heart of the patient; determine, at least based on the far-field sense signal and the local sense signal, a pulse delivery time and a pulse deliverable time window; and transmit, when the pulse delivery time falls into the pulse deliverable time window, the electrical stimulation pulse to the pair of stimulation electrodes.

29. A non-volatile computer-readable storage medium having a computer program stored thereon, wherein the computer program is executed by a processor to control an implantable device to perform a heart failure treatment method, wherein the implantable device comprises a pair of stimulation electrodes configured to be in contact with a predetermined stimulation position of the heart of a patient; and the method comprises:

sensing, by the pair of stimulation electrodes, a local sense signal generated by discharging of cardiac muscle cells at the predetermined stimulation position;
receiving a far-field sense signal indicating a global excitation event of the heart of the patient;
determining, at least based on the far-field sense signal and the local sense signal, whether a local excitation event of the cardiac muscle cells at the predetermined stimulation position corresponds to a specific global excitation event; and
when it is determined that the local excitation event corresponds to the specific global excitation event,
applying the electrical stimulation pulse to the predetermined stimulation position by using the pair of stimulation electrodes during an absolute refractory period of the local excitation event.

30. A non-volatile computer-readable storage medium having a computer program stored thereon, wherein the computer program is executed by a processor to control an implantable device to perform a heart failure treatment method, wherein the implantable device comprises a pair of stimulation electrodes configured to be in contact with a predetermined stimulation position of a heart of a patient; and the method comprises:

applying, by the pair of stimulation electrodes, an electrical pacing pulse for adjusting a heart rate of the heart of the patient to the predetermined stimulation position of the heart of the patient;
determining capture of the heart of the patient;
after the capture has been determined, determining, based on an obtained far-field sense signal indicating a global excitation event of the heart of the patient and the electrical pacing pulse, whether a local excitation event of cardiac muscle cells at the predetermined stimulation position corresponds to a specific global excitation event; and
when it is determined that the local excitation event corresponds to the specific global excitation event, applying the electrical stimulation pulse to the predetermined stimulation position by using the pair of stimulation electrodes during an absolute refractory period of the local excitation event.

FIG. 1

FIG. 2A

FIG. 2B

FIG. 2C

FIG. 3A

FIG. 3B

FIG. 3C

400a

408a 407a 411a 409a 410a

401a 404a 402a 403a

FIG. 4A

400b

407b 404b (411b) 409b 410b

401b 408b 402b 403b

FIG. 4B

400c

408c 407c 404c (411c) 402c

401c 409c 410c 403c

FIG. 4C

500a

501a    503a    504a

FIG. 5A

500b

501b    503b    504b

FIG. 5B

600

```
┌ ─ ─ ─ ─ ─ ─ ─ ─ ─ ─ ┐
│                                                    │   601
│ Determine an electrical signal source causing │
│         a local excitation event              │
│                                                    │
└ ─ ─ ─ ─ ─ ─ ─ ─ ─ ─ ┘
```

602

```
┌──────────────────────┐
│ Determine, at least based on an obtained │
│ global sense signal and local sense signal, │
│ whether a local excitation event of cardiac │
│ muscle cells at a predetermined stimulation │
│ position corresponds to a specific event in the │
│         global sense signal              │
└──────────────────────┘
```

604

```
┌──────────────────────┐
│ When it is determined that the local │
│ excitation event corresponds to the specific │
│ event in the global sense signal, transmit an │
│ electrical stimulation pulse to a pair of │
│ stimulation electrodes during an absolute │
│ refractory period of the local excitation event │
└──────────────────────┘
```

FIG. 6

FIG. 7

FIG. 8

| | |
|---|---|
| Acquiring a first sensing time of an R-wave in a preset electrocardiogram | S101 |
| Determining a sensing event in a local myocardial electrocardiogram corresponding to a set myocardial position based on the first sensing time, and acquiring a second sensing time corresponding to the sensing event | S102 |
| Determining that the sensing event is not an R-wave signal corresponding to the R-wave in the preset electrocardiogram if the second sensing time does not meet a first preset condition, and then not delivering pulse stimulation to a stimulation electrode at the set myocardial position | S103 |

FIG. 9

| S101 |
| Acquiring a first sensing time of an R-wave in a preset electrocardiogram |

| S102 |
| Determining a sensing event in a local myocardial electrocardiogram corresponding to a set myocardial position based on the first sensing time, and acquiring a second sensing time corresponding to the sensing event |

| S1031 |
| Acquiring a sensing time window corresponding to the R-wave in the preset electrocardiogram |

| S1032 |
| S103 |
| Determining that the sensing event is not the R-wave signal corresponding to the R-wave in the preset electrocardiogram if the second sensing time does not fall within the sensing time window, and then not delivering the pulse stimulation to the stimulation electrode at the set myocardial position |

## FIG. 10

| S101 |
| Acquiring a first sensing time of an R-wave in a preset electrocardiogram |

| S1021 |
| Taking the first sensing time as a first time point, and taking a time point from corresponding first set duration before the first time point as a time reference starting point |

| S1022 |
| S102 |
| Acquiring the sensing event in the local myocardial electrocardiogram corresponding to the set myocardial position based on the time reference starting point |

| S1031 |
| Acquiring a sensing time window corresponding to the R-wave in the preset electrocardiogram |

| S1032 |
| S103 |
| Determining that the sensing event is not the R-wave signal corresponding to the R-wave in the preset electrocardiogram if the second sensing time does not fall within the sensing time window, and then not delivering the pulse stimulation to the stimulation electrode at the set myocardial position |

## FIG. 11

| S101 |
| --- |
| Acquiring a first sensing time of an R-wave in a preset electrocardiogram |

| S102 |
| --- |
| Determining a sensing event in a local myocardial electrocardiogram corresponding to a set myocardial position based on the first sensing time, and acquiring a second sensing time corresponding to the sensing event |

| S104 |
| --- |
| Determining the sensing event as an R-wave if the second sensing time falls within the sensing time window |

## FIG. 12

| S105 |
| --- |
| Determining, after the sensing event is determined as an R-wave, a pulse delivery time corresponding to the stimulation electrode at the set myocardial position based on the second sensing time of the R-wave in the local myocardial electrocardiogram |

Determining whether the pulse delivery time meets a second preset condition

No

Yes

S106

Determining to deliver the pulse stimulation to the stimulation electrode at the set myocardial position

Not delivering the pulse stimulation to the stimulation electrode at the set myocardial position

## FIG. 13

Determining, after the sensing event is determined as an R-wave, a pulse delivery time corresponding to the stimulation electrode at the set myocardial position based on the second sensing time of the R-wave in the local myocardial electrocardiogram

S105

Acquiring a deliverable pulse time window corresponding to the R-wave in the preset electrocardiogram

S1061

Controlling the pulse stimulation to be delivered to the stimulation electrode at the set myocardial position in the pulse delivery time if the pulse delivery time falls within the deliverable pulse time window; and then not delivering the pulse stimulation to the stimulation electrode at the set myocardial position if the pulse delivery time does not fall within the deliverable pulse time window

S1062

S106

FIG. 14

FIG. 15

FIG. 16

First sensing time
acquisition module — 1

Sensing event determination
module — 2

Second sensing time
acquisition module — 3

First judgment module — 4

Control module — 5

Pulse stimulation control apparatus

FIG. 17

FIG. 18

FIG. 19

## INTERNATIONAL SEARCH REPORT

| International application No. |
| --- |
| **PCT/CN2023/096504** |

**A.    CLASSIFICATION OF SUBJECT MATTER**

A61N1/362(2006.01)i

According to International Patent Classification (IPC) or to both national classification and IPC

**B.    FIELDS SEARCHED**

Minimum documentation searched (classification system followed by classification symbols)

IPC: A61N

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 不应期, 局部, 心脏收缩力, 远场, 局部, 近场, 刺激, 电脉冲, 壳体, 锚定, 脉冲, refractory period, pulse, electronic, CCM, cardiac contractility modulation

**C.    DOCUMENTS CONSIDERED TO BE RELEVANT**

| Category* | Citation of document, with indication, where appropriate, of the relevant passages | Relevant to claim No. |
| --- | --- | --- |
| A | CN 110461411 A (CARDIAC PACEMAKERS, INC.) 15 November 2019 (2019-11-15) claims 1-15, and description, paragraphs [0106]-[0199] | 1-30 |
| A | CN 107072581 A (MEDTRONIC INC.) 18 August 2017 (2017-08-18) entire document | 1-30 |
| A | US 2016193467 A1 (BIOTRONIK SE & CO. KG) 07 July 2016 (2016-07-07) entire document | 1-30 |

☐ Further documents are listed in the continuation of Box C.          ☑ See patent family annex.

| | |
| --- | --- |
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **19 July 2023** | **12 September 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

INTERNATIONAL SEARCH REPORT
Information on patent family members

International application No.

**PCT/CN2023/096504**

| Patent document cited in search report | | | Publication date (day/month/year) | Patent family member(s) | | | Publication date (day/month/year) |
|---|---|---|---|---|---|---|---|
| CN | 110461411 | A | 15 November 2019 | JP | 2020511248 | A | 16 April 2020 |
| | | | | EP | 3600536 | A1 | 05 February 2020 |
| | | | | WO | 2018175314 | A1 | 27 September 2018 |
| | | | | AU | 2018237050 | A1 | 12 September 2019 |
| | | | | US | 2018264262 | A1 | 20 September 2018 |
| CN | 107072581 | A | 18 August 2017 | US | 2019351246 | A1 | 21 November 2019 |
| | | | | US | 2016106991 | A1 | 21 April 2016 |
| | | | | EP | 3209378 | A1 | 30 August 2017 |
| | | | | WO | 2016064577 | A1 | 28 April 2016 |
| US | 2016193467 | A1 | 07 July 2016 | EP | 3042694 | A1 | 13 July 2016 |

Form PCT/ISA/210 (patent family annex) (July 2022)